(19) Europäisches Patentamt
European Patent Office
Office européen des brevets

(11) EP 2 165 639 A1

(12) **EUROPEAN PATENT APPLICATION**
published in accordance with Art. 153(4) EPC

(43) Date of publication:
**24.03.2010 Bulletin 2010/12**

(21) Application number: **08765030.5**

(22) Date of filing: **03.06.2008**

(51) Int Cl.:
*A61B 1/00* (2006.01)     *A61B 1/04* (2006.01)
*A61B 5/07* (2006.01)

(86) International application number:
**PCT/JP2008/060217**

(87) International publication number:
**WO 2008/152947 (18.12.2008 Gazette 2008/51)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **12.06.2007 JP 2007155547**

(71) Applicant: **Olympus Corporation
Tokyo 151-0072 (JP)**

(72) Inventors:
• **NAGASE, Ayako
Tokyo 151-0072 (JP)**
• **SHIGEMORI, Toshiaki
Tokyo 151-0072 (JP)**

(74) Representative: **Schmidt, Steffen
Wuesthoff & Wuesthoff
Patentanwälte
Schweigerstrasse 2
81541 München (DE)**

(54) **IN-VIVO INFORMATION ACQUIRING DEVICE**

(57) An object of the invention is to permit the in-vivo information on subjects obtained by an in-subject inspection to be stored by specific information of the subjects and to smoothly execute the in-subject inspection to the subjects. An in-vivo information acquiring device 1 according to the invention includes a capsule endoscope 2 for capturing a group of in-vivo images and a receiving device 10 for receiving the group of the in-vivo images from the capsule endoscope 2. The capsule endoscope 2 has an imaging unit 4 for obtaining a specific image including the specific information of a subject and capturing a group of in-vivo images of the subject. The receiving device 10 has a storage unit 16 for storing the group of the in-vivo images captured by the imaging unit 4 and a control unit 18. The control unit 18 registers specific information included in the specific image and controls the storage unit 16 so that it causes the registered specific information to correspond to a group of in-vivo images of a subject specified by the specific information so as to store the group of in-vivo images of the subjects by specific information.

FIG.1

EP 2 165 639 A1

**Description**

TECHNICAL FIELD

The invention relates to an in-vivo information acquiring device for acquiring in-vivo information of a subject such as an image and the like obtained by capturing inside of an organ of the subject.

BACKGROUND ART

Recently, there is available, in a field of an endoscope, an in-vivo information acquiring device composed of a combination of a capsule endoscope, which has an imaging function and a radio communication function in a capsule-shaped casing, and a receiving device having an image data storage function. After the capsule endoscope of the in-vivo information acquiring device is swallowed from a mouth of a subject such as a patient and the like for observation (inspection), it sequentially captures images inside of the organs (which may be also called in-vivo images below) of the subject at predetermined intervals and sequentially wirelessly transmits the in-vivo images while moving in the organs such as a stomach, a small intestine, and the like by a peristaltic movement during a period of time until it is naturally discharged from the subject.

In contrast, the receiving device of the in-vivo information acquiring device is attached to the subject into organs of which the capsule endoscope is introduced, sequentially receives the in-vivo images wirelessly transmitted from the capsule endoscope, and accumulates a group of the in-vivo images received from the capsule endoscope in a storage unit (refer to, for example, Patent Document 1).

The group of the in-vivo images obtained by the in-vivo information acquiring device is captured by a workstation having an image display function and the like. The workstation displays the group of the in-vivo images captured from the receiving device on a display. A user such a doctor, a nurse, and the like diagnoses the subject by observing the in-vivo images displayed on the display of the work station.

Patent Document 1 : Japanese Patent Application Laid-open No. 2005-296186

DISCLOSURE OF THE INVENTION

PROBLEMS TO BE SOLVED BY THE INVENTION

Incidentally, in the receiving device described above, specific information such as a patient ID for specifying a patient and th like is previously registered in the receiving device before it receives an in-vivo image of a subject captured by the capsule endoscope, and the receiving device is attached to a subject which is specified by the registered specific information. The receiving device accumulates the group of the in-vivo images received from the capsule endoscope in the storage unit as the group of the in-vivo images of the subject which is specified by the specific information in the state that the specific information is registered.

However, when an in-subject inspection (which may be simply called an inspection below), which obtains in-vivo information such as an in-vivo image and the like by introducing the capsule endoscope into organs of the subject, is executed to a plularity of spicimens, the receiving device described above must cause the workstation and the like to capture the received group of the in-vivo images as well as must be initialized so that it can receive a group of in-vivo images of a next subject each time an in-subject inspection of one subject is completed, that is, each time a group of in-vivo images of one subject is received. Thus, each time an in-subject inspection to one subject is completed, a series of jobs must be repeated to remove the receiving device from a subject the inspection of which is completed, to carry the removed receiving device to a place such as a doctor's office and the like where the workstation is installed, to connect the receiving device to the workstation, and to cause the workstation to capture a group of in-vivo images. As a result, a problem arises in that when the in-subject inspection is executed to a plurality of subjects, a lot of troublesome jobs are required.

The invention was made in view of the above circumstances, and an object of the invention is to provide an in-vivo information acquiring device which can store in-vivo information of a plurality of subjects acquired by the in-subject inspection in each specific infomation of the spcimens so that the in-subject inspection can be smoothly executed to the subjects.

MEANS FOR SOLVING PROBLEM

To solve the problem described above and to achieve the object, an in-vivo information acquiring device according to the present invention is for acquiring in-vivo information on a plurality of subjects. The device includes an information acquiring unit configured to acquire specific information for specifying the subjects; a storage unit configured to store therein the in-vivo information on the subjects; and a control unit configured to execute control of registering the specific information acquired by the information acquiring unit in the storage unit and causing the registered specific information to correspond to the in-vivo information on a subject specified by the specific information so as to store the in-vivo information on the subjects by the specific information.

In the in-vivo information acquiring device according to the present invention as set forth in the invention described above, the information acquiring unit is an imaging unit for capturing a specific image including the specific information, and the control unit executes control of registering the specific information included in the specific image captured by the imaging unit in the storage unit and causing the registered specific information to correspond to the in-vivo information on a subject specified by the specific information so as to store the in-vivo information on the subjects by the specific information.

In the in-vivo information acquiring device according to the present invention as set forth in the invention described above, the in-vivo information is an in-vivo image obtained by imaging inside of an organ of the subject, and the imaging unit images the specific image and further images the in-vivo image.

In the in-vivo information acquiring device according to the present invention as set forth in the invention described above, the specific image is living body information of the subject.

In the in-vivo information acquiring device according to the present invention as set forth in the invention described above, the information acquiring unit is a receiving unit for receiving the specific information by executing a radio communication, and the control unit executes control of registering the specific information received by the receiving unit and causing the registered specific information to correspond to the in-vivo information on a subject specified by the specific information so as to store the in-vivo information on the subjects by the specific information.

In the in-vivo information acquiring device according to the present invention as set forth in the invention described above, the information acquiring unit is an input unit for inputting the specific information to the control unit, and the control unit executes control of registering the specific information input by the input unit to the storage unit and causing the registered specific information to correspond to the in-vivo information on a subject specified by the specific information so as to store the in-vivo information on the subjects by the specific information.

The in-vivo information acquiring device according to the present invention as set forth in the invention described above further includes a display unit for displaying register request information for requesting to newly register the specific information. The control unit executes a control for displaying the register request information before newly registering the specific information in the storage unit.

In the in-vivo information acquiring device according to the present invention as set forth in the invention described above, the control unit determines, for each piece of the in-vivo information, whether acquisition of the in-vivo information to be stored in the storage unit has been finished and causes, during a period from a time at which specific information is newly registered in the storage unit to a time at which the control unit determines that the acquisition of the in-vivo information of a subject specified by the newly registered specific information has been finished, a series of the in-vivo information stored in the storage unit to correspond to the newly registered specific information.

The in-vivo information acquiring device according to the present invention as set forth in the invention described above further includes an instruction unit for instructing to finish to store the in-vivo information on the subject. When the instruction unit instructs to finish the storage, the control unit determines that the acquisition of the in-vivo information of the subject specified by the newly registered specific information has been finished and causes, during a period from a time at which specific information is newly registered in the storage unit to a time at which the instruction unit instructs to finish the storage, a series of the in-vivo information stored in the storage unit to correspond to the newly registered specific information.

In the in-vivo information acquiring device according to the present invention as set forth in the invention described above, the specific image is an optical information recording medium on which optical information corresponding to the subject is recorded.

In the in-vivo information acquiring device according to the present invention as set forth in the invention described above, the control unit determines that acquisition of the in-vivo information of a subject specified by the newly registered specific information has been finished in an arbitrarily set period of time after specific information is newly registered in the storage unit and causes a series of in-vivo information stored in the storage unit to correspond to the newly registered specific information during a period until the determination is made.

In the in-vivo information acquiring device according to the present invention as set forth in the invention described above, the control unit determines that acquisition of the in-vivo information on a subject specified by specific information has been finished based on at least one of color information, luminance information, and space-frequency information of the in-vivo information of a subject specified by the newly registered specific information after specific information is registered in the storage unit, and causes a series of in-vivo information stored in the storage unit to correspond to the newly registered specific information during a period until the determination is made.

In the in-vivo information acquiring device according to the present invention as set forth in the invention described above, the control unit determines, by inputting the in-vivo information of a subject specified by the newly registered specific information from the outside after specific information is registered in the storage unit, that acquisition of the in-vivo information on a subject specified by specific information has been finished, and causes a series of in-vivo information stored in the storage unit to correspond to the newly registered specific information during a period until the determination is made.

EFFECT OF THE INVENTION

According to the in-vivo information acquiring device of the invention, in-vivo information of a plurality of subjects can be stored while maintaining a state that the in-vivo images can be identified by specific information of the subjects, and the in-vivo information of the subjects stored by the specific information can be collectively downloaded to an external device such as the workstation and the like. As a result, an effect can be achieved in that repetition of a series of troublesome jobs for causing the external device to capture in-vivo information of one subject each time the acquisition of the in-vivo information of the subject is finished can be omitted and that an in-subject inspection for acquiring in-vivo information of a subject can be smoothly executed to a plurality of subjects.

BRIEF DESCRIPTION OF DRAWINGS

FIG. 1 is a block diagram schematically showing a configuration example of an in-vivo information acquiring device according to an first embodiment of the invention.
FIG. 2 is a schematic view exemplifying a state that patient information for specifying a subject is obtained by an imaging unit.
FIG. 3 is a flowchart exemplifying an inspection procedure of an in-subject inspection executed to a plurality of subjects.
FIG. 4 is a flowchart exemplifying a processing procedure of a control unit disposed to a receiving device of the first embodiment.
FIG. 5 is a schematic view exemplifying a state that the in-subject inspection is executed to a plurality of subjects using the in-vivo information acquiring device according to the first embodiment.
FIG. 6 is a block diagram schematically showing a configuration example of an in-vivo information acquiring device according to a second embodiment of the invention.
FIG. 7 is a schematic view exemplifying an image signal including image data of an in-vivo image and the patient information of a subject.
FIG. 8 is a flowchart exemplifying a processing procedure of a control unit disposed to a receiving device of the second embodiment.
FIG. 9 is a schematic view exemplifying a state that the in-subject inspection is executed to a plurality of subjects using the in-vivo information acquiring device according to the second embodiment.
FIG. 10 is a block diagram schematically showing a configuration example of an in-vivo information acquiring device according to a third embodiment of the invention.
FIG. 11 is a schematic view exemplifying a state that the in-subject inspection is executed to a plurality of subjects using the in-vivo information acquiring device according to the third embodiment.
FIG. 12 is a block diagram schematically showing a configuration example of an in-vivo information acquiring device according to a modification of the third embodiment of the invention.

EXPLANATIONS OF LETTERS OF NUMERALS

| 1, 21, 41, 61 | In-vivo information acquiring device |
|---|---|
| 2, 22 | Capsule endoscope |
| 3 | Illuminating unit |
| 4 | Imaging unit |
| 5, | 13, 25, 33 Image processing unit |
| 6 | Transmitting unit |
| 6a | Transmitting antenna |
| 7, | 16 Storage unit |
| 8, | 18, 28, 38, 58, 78 Control unit |
| 9, | 19 Power supply unit |

| 10, 30, 50, 70 | Receiving device |
| 11 | Receiving unit |
| 11a to 11g | Receiving antenna |
| 12 | Signal detecting unit |
| 14 | Operating unit |
| 15 | Display unit |
| 17 | Output unit |
| 18a, 38a, 58a, 78a | Patient information processing unit |
| 18b | Display control unit |
| 18c, 38c | Storage control unit |
| 23 | RFID tag |
| 23a | Antenna |
| 25a | Addition processing unit |
| 33a | Information extracting unit |
| 57, 77 | Input unit |
| 77a | Input key group |
| 100 | Workstation |
| 101 | Cradle |
| 110 | RFID writer |
| 120 | Register device |
| A | Optical information recording medium |
| Fn | Folder |
| Kn | Specimen |

BEST MODES FOR CARRYING OUT THE INVENTION

Preferable embodiments of an in-vivo information acquiring device according to the invention will be explained below in detail referring to the drawings. Note that, although the embodiments of the invention will be explained exemprifying an in-vivo information acquiring device composed of a combination of a capsule endoscope, which is introduced into organs of a subject such a patient and the like and captures an in-vivo image, and a receiving device for receiving the in-vivo image, the invention is not limited by the embodiments.

First Embodiment

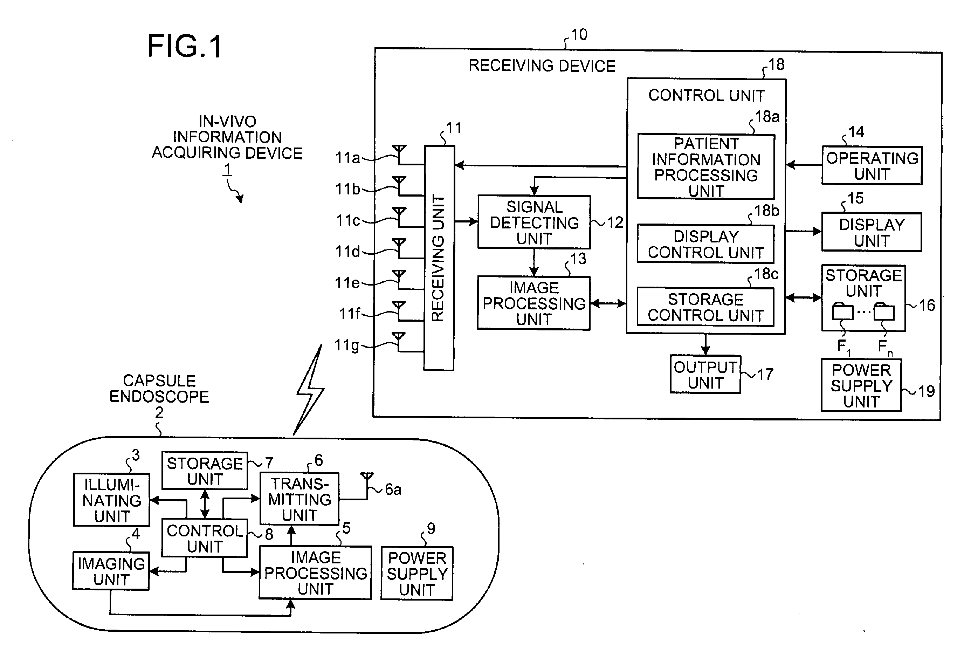

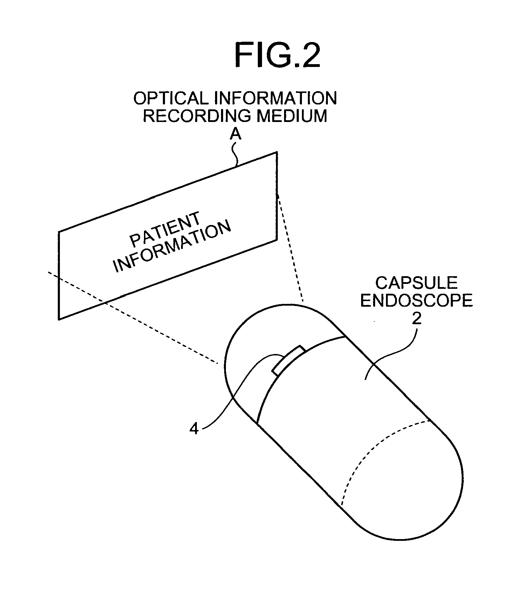

FIG. 1 is a block diagram schematically showing a configuration example of an in-vivo information acquiring device according to an first embodiment of the invention. FIG. 2 is a schematic view exemplifying a state that patient information for specifying a subject is acquired by an imaging unit. As shown in FIG. 1, an in-vivo information acquiring device 1 according to the first embodiment has a capsule endoscope 2 for capturing an in-vivo image (an example of in-vivo information) of a subject and a receiving device 10 for receiving in-vivo images captured by the capsule endoscope 2 and sequentially accumulating the received in-vivo images in a storage unit. A configuration of the capsule endoscope 2 will be explained first and then a configuration of the receiving device 10 will be explained below referring to FIGS. 1 and 2.

The capsule endoscope 2 has an imaging function and a radio communication function in a capsule-shaped casing formed in a size which can be easily introduced into organs of a subject such as a patient and the like. Specifically, when an in-subject inspection is executed to obtain an in-vivo image of a subject, the capsule endoscope 2 first captures an image including patient information for specifying the subject (hereinafter, called a specific image) and wirelessly transmits the captured specific image to the receiving device 10. Thereafter, the capsule endoscope 2 is introduced into organs of the subject and sequentially captures in-vivo images of the subject at predetermined intervals (intervals of, for example, 0.25 second) while moving in the organs by a peristaltic movement and the like. Then, the capsule endoscope 2 wirelessly transmits the in-vivo images to the receiving device 10 sequentially. The capsule endoscope 2 includes an illuminating unit 3, an imaging unit 4, an image processing unit 5, a transmitting unit 6, a storage unit 7, a control unit 8, and a power supply unit 9 in the capsule-shaped casing.

The illuminating unit 3 includes a plurality of light emitting devices such as LEDs and the like and a drive circuit for driving the light emitting devices. The illuminating unit 3 emits predetermined illuminating light to an image capturing field of view of the imaging unit 4 to thereby illuminate the image capturing field of view of the imaging unit 4.

The imaging unit 4 acts as an imaging means for capturing an in-vivo image of a subject as well as acts as an information acquiring means for acquiring the patient information of the subject. Specifically, the imaging unit 4 includes a solid imaging device such as a CCD or CMOS imaging sensor and the like and an optical system such as lenses and the like for imaging an optical image of a subject on a light receiving surface of the solid imaging device. The imaging unit 4 receives light reflected from the image capturing field of view illuminated by the illuminating unit 3 and captures a subject image in the image capturing field of view (for example, an in-vivo image or a specific image). Each time the imaging unit 4 captures the subject image, it transmits obtained image data to the image processing unit 5.

The imaging unit 4 obtains the patient information of a subject before the capsule endoscope 2 is introduced into organs of the subject. In this case, the imaging unit 4 captures a specific image including patient information recorded on an optical information recording medium A as shown in, for example, FIG. 2. The optical information recording medium A has optical information in which the patient information of subjects is one-dimensionally or two-dimensionally coded or optical information in which the patient information of subjects is recorded in a visible mode (for example, by alphameric characters, characters, and the like). The optical information can be captured by the imaging unit 4 as information showing the patient information of the subjects. The imaging unit 4 can obtain the patient information recorded on the optical information recording medium A as the optical information by capturing a specific image including the patient information recorded in the optical information recording medium A. The imaging unit 4 transmits image data of the specific image to the image processing unit 5. Note that the patient information of the subjects is spcific information for specifying each of the subjects and includes, for example, a patient ID, a patient name, a birth day, a gender, and the like.

When the capsule endoscope 2 is introduced into organs of a subject, the imaging unit 4 captures in-vivo images of the subject. Specifically, the imaging unit 4 sequentially captures the in-vivo images of the subject at predetermined intervals (at intervals of, for example, 0.25 second) by receiving light reflected from inside of the internal organs illuminated by the illuminating unit 3. Each time the imaging unit 4 captures the in-vivo image, it sequentially transmits the obtained image data to the image processing unit 5.

The image processing unit 5 obtains the image data of a subject image captured by the imaging unit 4 therefrom and creates an image signal including the subject image based on the obtained image data. Specifically, when the imaging unit 4 captures a specific image, the image processing unit 5 executes a predetermined image processing on the image data received from the imaging unit 4 and creates the image signal including the specific image. Further, when the imaging unit 4 captures an in-vivo image, the image processing unit 5 executes a predetermined image processing on image data received from the imaging unit 4 and creates the image signal including the in-vivo image. Each time the image processing unit 5 creates the image signal including the specific image or the in-vivo image, it sequentially transmits the created image signal to the transmitting unit 6. Note that the image signal created by the image processing unit 5 includes a synchronizing signal, inherent identification parameters such as a capsule ID for specifying the capsule endoscope 2 and the like, image processing parameters such as a white balance and the like in addition to the specific image or the in-vivo image described above.

The transmitting unit 6 has, for example, a coil-shaped transmitting antenna 6a and wirelessly transmits the specific image and the in-vivo image described above to the outside through the transmitting antenna 6a. Specifically, the transmitting unit 6 executes a predetermined wireless transmission process such as a modulation process and the like to the image signal obtained from the image processing unit 5 and creates a radio signal corresponding to the image signal. The transmitting unit 6 sequentially transmits the radio signal to the external receiving device 10 through the transmitting antenna 6a. The radio signal transmitted by the transmitting unit 6 includes the specific information or the in-vivo image captured by the imaging unit 4 described above, the synchronizing signal, the inherent identification parameters such as the capsule ID of the capsule endoscope 2 and the like, the various image processing parameters, and the like.

The storage unit 7 stores therein the inherent identification parameters such as the capsule ID and the like for specifying the capsule endoscope 2 and the various image processing parameters such as the white balance and the like. The capsule ID and the image processing parameters stored in the storage unit 7 are read out by the control unit 8 and wirelessly transmitted to the external receiving device 10 together with the specific image or the in-vivo image as described above.

The control unit 8 controls the respective units (the illuminating unit 3, the imaging unit 4, the image processing unit 5, the transmitting unit 6, and the storage unit 7) of the capsule endoscope 2 and controls signals input and output between the respective units. Specifically, the control unit 8 controls operation timings of the illuminating unit 3 and the imaging unit 4 so that the imaging unit 4 captures a subject image (the specific image or the in-vivo image) in the image capturing field of view illuminated by the illuminating unit 3. Further, the control unit 8 controls the image processing unit 5 so that it creates an image signal including the specific image or the in-vivo image, the synchonizing signal, the inherent identification parameters such as the capsule ID and the like, and the various image processing parameters, and the like described above. The control unit 8 controls the transmitting unit 6 so that it creates a radio signal corresponding to the image signal created by the image processing unit 5 and controls the transmitting unit 6 so that it transmit the radio signal to the external receiving device 10.

The power supply unit 9 supplies power to the respective units of the capsule endoscope 2. Specifically, the power supply unit 9 includes a button battery having predetermined power and a switch circuit turned on and off by a magnetic force from the outside. When the switch circuit is turned on by the magnetic force from the outside, the power supply unit 9 supplies power to the respective units (the illuminating unit 3, the imaging unit 4, the image processing unit 5, the transmitting unit 6, the storage unit 7, and the control unit 8) of the capsule endoscope 2.

Next, a configuration of the receiving device 10 of the in-vivo information acquiring device 1 according to the first embodiment will be explained. As shown in FIG. 1, the receiving device 10 includes a receiving unit 11, a signal detecting unit 12, and an image processing unit 13. Further, the receiving device 10 includes an operating unit 14, a display unit 15, a storage unit 16, an output unit 17, a control unit 18, and a power supply unit 19. Specifically, the receiving device 10 is worn on a subject into organs of which the capsule endoscope 2 is introduced and receives a specific image including the patient information of the subject from the capsule endoscope 2.

In this case, the receiving device 10 registers the patient information included in the in-vivo image. Thereafter, the receiving device 10 sequentially receives in-vivo images which are wirelessly transmitted from the capsule endoscope 2 introduced into the organs of the subject and accumulates a group of the in-vivo images of the subject in the storage unit after causing the group of the in-vivo images to correspond to the registered patient information. Finally, the receiving device 10 accumulates groups of in-vivo images of a plurality of subjects as targets of the in-subject inspection in the storage unit by patient information.

The receiving unit 11 acts as a receiving means for receiving the radio signal transmitted by the transmitting unit 6 of the capsule endoscope 2 described above. Specifically, the receiving unit 11 has a plurality of receiving antennas 11a to 11g and receives the radio signal from the capsule endoscope 2 through the receiving antennas 11a to 11g. Further, the receiving unit 11 executes a predetermined receiving process such as a demodulation process and the like to the radio signal received through the receiving antennas 11a to 11g and demodulates the radio signal to an image signal. The image signal demodulated by the receiving unit 11 includes the specific image or the in-vivo image, the synchonizing signal, the inherent identification parameters such as the capsule ID of the capsule endoscope 2 and the like, the various image processing parameters, and the like as described above. The receiving unit 11 transmits the image signal to the signal detecting unit 12.

The receiving antennas 11a to 11g are dispersedly disposed on a body surface of the subject having the receiving device 10 worn thereon (carried thereby) and connected to the receiving unit 11 through connectors. The receiving unit 11 can receive the radio signal from the capsule endoscope 2 in the organs of the subject through the receiving antennas 11a to 11g. Note that it is sufficient that at least one antenna be connected to the receiving unit 11, and a number of the receiving antennas is not particularly limited to 7.

The signal detecting unit 12 detects an image signal (image signal per frame) including a specific image or an in-vivo image in one frame. Specifically, the signal detecting unit 12 sequentially obtains image signals demodulated by the receiving unit 11 and sequentially detects vertical synchronizing signals of the obtained image signals. The signal detecting unit 12 detects image signals, which are obtained until it detects a next vertical synchronizing signal after it detects one vertical synchronizing signal, as the image signal per frame. The signal detecting unit 12 sequentially transmits the image signals per frame detected as described above to the image processing unit 13.

The image processing unit 13 creates the specific image or the in-vivo image described above based on the image signal per frame detected by the signal detecting unit 12. Specifically, the image processing unit 13 sequentially obtains the image signals per frame from the signal detecting unit 12. The image signal per frame includes image data of the specific image or the in-vivo image of one frame, the synchonizing signal, the inherent identification parameters such as the capsule ID of the capsule endoscope 2 and the like, the image processing parameters such as the white balance etc., and the like. The image processing unit 13 creates a specific image of one frame using the image data of the specific image of one frame and the various image processing parameters. The image processing unit 13 also creates the in-vivo image of one frame using the image data of the in-vivo image of one frame and the various image processing parameters. Each time when the image processing unit 13 creates the specific image or the in-vivo image of one frame, it sequentially transmits the created specific image or in-vivo image, the capsule ID, and the like to the control unit 18.

The operating unit 14 is used to finish to store the in-vivo image captured by the capsule endoscope 2 introduced into the organs of the subject and acts as an instruction means for instructing the control unit 18 to finish to store the in-vivo image (an example of the in-vivo information of the subject). Specifically, the operating unit 14 has an operation button and the like for finishing to store the in-vivo image. The operating unit 14 instructs the control unit 18 to finish to store the in-vivo image by depressing the operation button.

The display unit 15 includes a liquid crystal display and the like and displays various information whose display is instructed by the control unit 18. The various information displayed on the display unit 15 includes, for example, the patient information of a current subject who wears the receiving device 10 (to which the in-subject inspection is being executed), and register request information for requesting the receiving device 10 to register new patient information, and the like.

The storage unit 16 acts as a storage means for storing groups of in-vivo images of a plurality of subjects by patient information. Specifically, the storage unit 16 sequentially stores the patient information of subjects whose register is instructed by the control unit 18. Each time the storage unit 16 newly stores (registers) therein the patient information, it adds folders $F_n$ (n = 1, 2, 3 ...) corresponding to the stored patient information. The storage unit 16 has folders $F_1$, ..., $F_n$ of respective pieces of the patient information stored sequentially. The folders $F_1$, ..., $F_n$ of the storage unit 16 are caused to correspond to the patient information of the respective subjects, respectively by setting, for example, the patient information of the respective subjects as folder names. The storage unit 16 stores groups of in-vivo images of subjects, which are specified by patient information that agrees with the folder names, in the folders $F_1$, ..., $F_n$, respectively. As described above, the storage unit 16 stores therein the groups of the in-vivo images of the subjects by patient information in a state that the patient information of the subjects are caused to correspond to the group of the in-vivo images of the subjects.

The output unit 17 acts as an information output interface for outputting the groups of the in-vivo images, which is stored in each patient information by the storage unit 16, to the outside. Specifically, the output unit 17 is connected to an external work station (not shown) for capturing the groups of the in-vivo images of the subjects and outputs a group of in-vivo images whose output is instructed by the control unit 18 to the external work station. The groups of the in-vivo images of the subjects in the storage unit 16 described above are captured by the external workstation through the output unit 17.

The control unit 18 controls the respective units of the receiving device 10 (the receiving unit 11, the signal detecting unit 12, the image processing unit 13, the operating unit 14, the display unit 15, the storage unit 16, and the output unit 17). Specifically, the control unit 18 controls operations of the receiving unit 11, the signal detecting unit 12, the image processing unit 13, the operating unit 14, the display unit 15, the storage unit 16, and the output unit 17 described above and controls signals input and output between the respective units. The control unit 18 registers patient information included in a specific image captured by the imaging unit 4 of the capsule endoscope 2 described above in the storage unit 16 and controls the storage unit 16 so that it stores the groups of the in-vivo images of the subjects by registered patient information. In this case, the control unit 18 causes a series of in-vivo images, which is obtained from the image processing unit 13 during a period from a time at which patient information is newly registered in the storage unit 16 to a time at which next patient information is registered in correspond to the newly registered patient information.

The control unit 18 has a patient information processing unit 18a, a display control unit 18b, and a storage control unit 18c. The patient information processing unit 18a extracts an specific image from a group of subject images (the specific image and the in-vivo image) sequentially transmitted by the image processing unit 13 to the control unit 18 based on color information of respective pixels, and the like. The patient information processing unit 18a extracts patient information included in the specific image and newly registers the extracted patient information in the storage unit 16. Each time the patient information processing unit 18a newly obtains the specific image created by the image processing unit 13, it newly registers the patient information included in the new specific image in the storage unit 16. Each time the patient information processing unit 18a newly registers the patient information extracted from the specific image in the storage unit 16, it creates folders $F_n$ (n = 1, 2 ...) which use the new patient information as folder names in the storage unit 16. The patient information processing unit 18a creates a plurality of the folders $F_1$, ..., $F_n$ in the storage unit 16 by patient information as described above.

Each time, for example, the receiving device 10 is started or each time the group of the in-vivo images of the subject specified by the patient information newly registered in the storage unit 16 has been obtained (stored), the display control unit 18b controls the display unit 15 so that it displays register request information for requesting to newly register the patient information of a subject. Thereafter, when the patient information processing unit 18a described above newly registers patient information in the storage unit 16, the display control unit 18b controls the display unit 15 so that it finishes to display the register request information. In this case, the display control unit 18b controls the display unit 15 so that it displays register finish information of the patient information for a predetermined time in place of the register request information. Note that the register finish information is information showing that the patient information of a subject is newly registered in the receiving device 10 (specifically, in the storage unit 16).

After the display control unit 18b causes the display unit 15 to display the register finish information for the predetermined time, it controls the display unit 15 so that it displays the newly registered patient information. With this operation, the display control unit 18b can cause the display unit 15 to display the patient information of the current subject on which the receiving device 10 is worn to execute an in-subject inspection from now.

The storage control unit 18c controls the storage unit 16 so that it stores therein the groups of the in-vivo images of the subjects by the patient information which is registered in the storage unit 16 by the patient information processing unit 18a described above. Specifically, the storage control unit 18c extracts an in-vivo image from a group of subject images (a specific image and an in-vivo image) sequentially transmitted to the control unit 18 by the image processing unit 13 based on color information of respective pixels, and the like. The storage control unit 18c controls the storage unit 16 so that it sequentially stores therein the in-vivo images obtained from the image processing unit 13 as described above by patient information. In this case, the storage control unit 18c determines, for each piece of the patient information, that the acquisition of the groups of the in-vivo images of the subjects to be stored in the storage unit 16 have been finished and causes a series of in-vivo images (i.e., the group of the in-vivo images captured by the capsule endoscope 2 in the organs of the current subject), which are stored in the storage unit 16 during a period from a time at which the patient information processing unit 18a newly registers the patient information of the current subject in the storage unit 16 to a time at which it is determined that the acquisition of the group of the in-vivo images of the current subject has been finished, to correspond to the patient information of the current subject that is the newly registered patient information.

More specifically, the storage control unit 18c specifies a new folder (i.e., a folder newly created using the patient information of the current subject as the new patient information as its folder name) of the folders $F_1$, ..., $F_n$ in the storage unit 16 as a folder, in which in-vivo images are newly stored, and stores the series of in-vivo images in the specified new folder. Further, the storage control unit 18c stores a capsule ID and the like obtained together with a specific image or an in-vivo image including the patient information of the current subject in the new folder. As described above, the storage control unit 18c causes the patient information of the current subject, the group of the in-vivo images of the current subject, and the capsule ID of the capsule endoscope 2 introduced into the organs of the current subject to correspond with each other.

The storage control unit 18c repeats the same process and the same control to a plurality of subjects and causes the storage unit 16 to store the groups of the in-vivo images of the subjects by patient information. In this case, the groups of the in-vivo images of the subjects, which are specified by the patient information as the folder names, capsule IDs, and the like are stored in the folders $F_n$ (n = 1, 2, ...) in the storage unit 16.

When the storage control unit 18c determines whether the acquisition of the group of the in-vivo images of the subject specified by the new patient information described above has been finished, it may determine it by using the fact, as a trigger, that the operating unit 14 instructs to finish storing or that a predetermined time has passed since the new patient information is registered in the storage unit 16 as a trigger. When the operating unit 14 instructs to finish storing, the storage control unit 18c forcibly finishes a process for storing the group of the in-vivo images of the subject specified by the new patient information in the new folder.

Further, when predetermined time information is previously set and a time corresponding to the time information has passed since patient information is newly registered in the storage unit 16, the storage control unit 18c finishes the process for storing the group of the in-vivo images of the subject specified by the new patient information in the new folder. In this case, when the time information is set in conformity with a moving time of the capsule endoscope 2 moving in organs, the storage control unit 18c can store a group of in-vivo images (i.e., a group of in-vivo images in desired portions) captured by the capsule endoscope 2 while moving inside of desired organs such as a stomach, a small intestine, and the like in the storage unit 16 by causing the group of in-vivo images to correspond to the new patient information.

The power supply unit 19 supplies power to the respective units of the receiving device 10. Specifically, the power supply unit 19 includes a battery having predetermined power and a power supply switch. When the power supply switch is turned on, the power supply unit 19 supplies power to the respective units of the receiving device 10 (the receiving unit 11, the signal detecting unit 12, the image processing unit 13, the operating unit 14, the display unit 15, the storage unit 16, the output unit 17, and the control unit 18). In this case, the receiving device 10 is started.

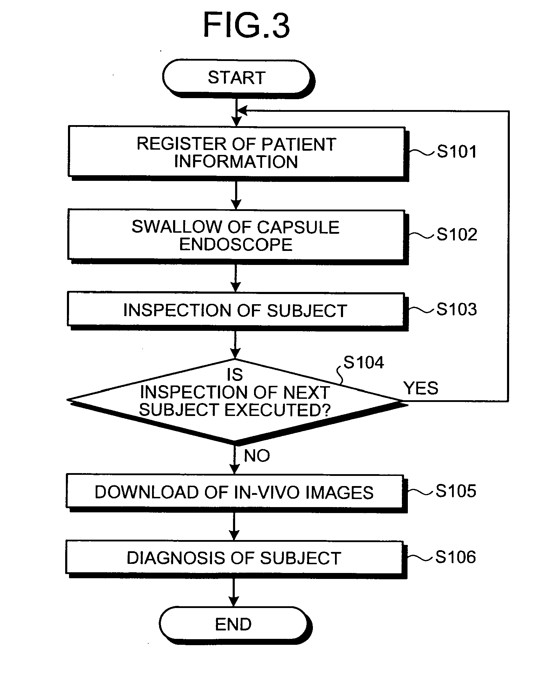

Next, a job procedure of an in-subject inspection executed to a plurality of subjects to obtain groups of in-vivo images of the subjects will be explained. FIG. 3 is a flowchart exemplifying an inspection procedure of the in-subject inspection to the subjects.

As shown in FIG. 3, first, a user such as a doctor, a nurse, or the like registers the patient information of a subject to the receiving device 10 (step S101). Specifically, the user causes the subject as an inspection target to wear the receiving device 10. Note that the receiving antennas 11a to 11g are dispersingly disposed on a body surface of the subject previously. The user connects the receiving unit 11 of the receiving device 10 worn on the subject to the receiving antennas 11a to 11g. In this state, the user starts the receiving device 10 by turning on the power supply unit of the receiving device 10. Then, the user registers patient information to the receiving device 10 according to register request information displayed on the display unit 15 of the receiving device 10.

When the patient information is registered in the receiving device 10, the user first captures a specific image including the patient information of the subject using the capsule endoscope 2 to be swallowed by the subject. Specifically, as shown in FIG. 2, the user places the optical information recording medium A in an image capturing field of view of the capsule endoscope 2 and causes the capsule endoscope 2 to capture optical information (i.e., the patient information of the subject) recorded on the optical information recording medium A. In this case, the capsule endoscope 2 captures a specific image including the patient information of the subject and transmits a radio signal including the captured specific image to the outside. The receiving device 10 worn on the subject receives the radio signal transmitted from the capsule endoscope 2 and obtains the specific image included in the received radio signal. The receiving device 10 obtains the patient information included in the specific image, i.e., the patient information of the subject and registers therein the obtained patient information.

Next, the user causes the subject carrying the receiving device 10 to which the patient information is registered in swallow the capsule endoscope 2 (step S102) and starts an inspection of the subject, i.e., an in-subject inspection for obtaining a group of in-vivo images of the subject (step S103).

In the in-subject inspection, the capsule endoscope 2 is swallowed from a mouth of the subject and introduced into organs of the subject. The capsule endoscope 2 sequentially captures in-vivo images at predetermined intervals (intervals of, for example, 0.25 second) while moving inside of the organs of the subject (an esophagus, a stomach, a small intestine, and the like) and sequentially transmits a radio signal including the obtained in-vivo images to the outside. Each time the receiving device 10 worn on the subject receives the radio signal transmitted from the capsule endoscope 2 in the organs, it obtains the in-vivo images included in the received radio signal. Then, the receiving device 10 causes a series of the in-vivo images sequentially obtained from the capsule endoscope 2 in the organs to correspond to the patient information (i.e., the patient information of the subject) registered at step S101 and stores a group of the in-vivo images of the subject in the storage unit 16.

The inspection to the subject is finished when a predetermined time has passed after the patient information is registered in the receiving device 10 at step S101. The predetermined time is a time corresponding to the time information previously set to the storage control unit 18c of the receiving device 10 and set in conformity with a moving time of the capsule endoscope 2 in the organs of the subject as described above. With this in-subject inspection, a group of in-vivo images inside of a desired organ, for example, a partial desired portion of a stomach, a small intestine, a large intestine, or the like of the subject or a group of in-vivo images of all the portions of a gastrointestinal tract from a mouth cavity to the large intestine through an esophagus, the stomach, and the small intestine can be stored in the storage unit 16 of the receiving device 10.

Thereafter, the user removes the receiving device 10 worn on the subject. In this case, the group of the in-vivo images (i.e., the group of the in-vivo images of the subject), which is caused to correspond to the patient information of the subject, is stored in the receiving device 10. When the user inspects a next subject following the inspected subject (step S104: Yes), the user causes the next subject to wear the removed receiving device 10 and connects the receiving device 10 to the receiving antennas 11a to 11g dispersingly disposed on a body surface of the subject. Thereafter, the user repeats the inspection procedure at step S101 and subsequent steps.

The user executes the in-subject inspection to a plurality of subjects from which groups of in-vivo images are obtained by repeating the inspection procedure of steps S101 to S104 a necessary number of times. When the user does not inspect the next subject following the inspected subjects after he or she executes the inspection to a desired number of subjects as described above (step S104, No), the user removes the receiving device 10 worn on a final subject in the in-subject inspection and downloads the groups of the in-vivo images stored in the removed receiving device 10 to the workstation and the like (step S105).

At step S105, the user connects the receiving device 10 removed from the subject to the workstation through a cradle and the like. Note that the groups of the in-vivo images of the subjects to which the in-subject inspection is executed are stored in the receiving device 10 by patient information. The user causes the workstation to collectively download the groups of the in-vivo images of the subjects stored in the receiving device 10 by a download job executed once. As a result, the workstation can capture the groups of the in-vivo images of the subjects from the receiving device 10 at a time. The groups of the in-vivo images of the subjects are caused to correspond to their respective patient information of the subjects. Therefore, the workstation causes the groups of the in-vivo images of the subjects captured from the receiving device 10 to correspond to the patient information of the respective subjects and holds and manages the groups of the in-vivo images of the subjects by patient information.

Thereafter, the user diagnoses a desired subject based on the group of the in-vivo images downloaded to the workstation (step S106). In this case, the user operates the workstation, which captures the groups of the in-vivo images of the subjects, and displays the group of the in-vivo images of a desired subject of these subjects on the display of the work station. The user diagnoses the desired subject by observing the group of the in-vivo images displayed on the workstation.

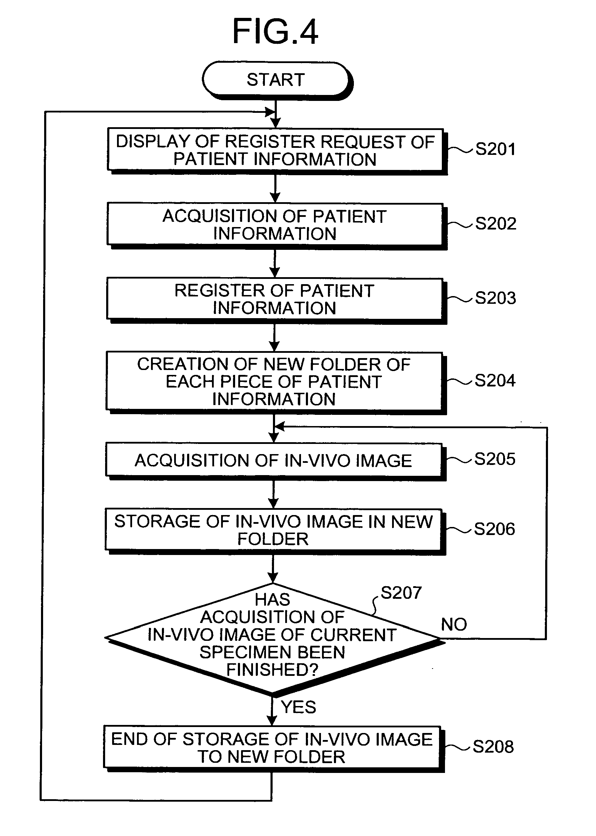

Next, an operation of the control unit 18 of the receiving device 10 for storing the groups of the in-vivo images of a plurality of subjects by patient information when the in-subject inspection described above is executed to the subjects will be explained. FIG. 4 is a flowchart exemplifying a processing procedure of the control unit 18 of the receiving device 10 of the first embodiment.

As shown in FIG. 4, first, the control unit 18 causes the display unit 15 to display a register request of the patient information of a subject (step S201). Specifically, when the control unit 18 is started by turning on the power supply unit 19 (that is, when the receiving device 10 is started), it causes the display unit 15 to display the register request of the patient information of the subject. In this case, the display control unit 18b controls the display unit 15 so that it displays the register request information described above.

Next, the control unit 18 acquires the patient information of a current subject on which the receiving device 10 is worn to execute the in-subject inspection (step S202). Specifically, the imaging unit 4 of the capsule endoscope 2 captures the optical information recording medium A on which the patient information of the current subject is recorded according to the register request information displayed on the display unit 15 at step S201. As a result, the imaging unit 4 captures a specific image including the patient information of the current subject. The capsule endoscope 2, which captures the specific image by the imaging unit 4, transmits a radio signal including the specific image to the receiving device 10 worn on the current subject. The receiving unit 11 of the receiving device 10 receives the radio signal and demodulates it to an image signal, and the image processing unit 13 creates a specific image included in the image signal. The control unit 18 obtains the specific image created by the image processing unit 13. In this case, the patient information processing unit 18a extracts the specific image based on color information and the like of respective pixels and obtains the patient information of the current subject included in the extracted specific image.

Thereafter, the control unit 18 registers the patient information of the current subject obtained at step S202 (step S203) and newly creates a folder of each patient information using the registered patient information (step S204). In this case, the patient information processing unit 18a writes the patient information of the current subject in the storage unit 16 to thereby newly register the patient information of the current subject in the storage unit 16. Then, the patient information processing unit 18a newly creates a folder that uses the registered patient information as a folder name in the storage unit 16. The folder that uses the patient information of the current subject as the folder name is a new folder newly created in the storage unit 16.

When the patient information processing unit 18a newly registers the patient information in the storage unit 16, the display control unit 18b controls the display unit 15 so that it displays register finish information for a predetermined time in place of the register request information described above. After the predetermined time passes, the display control unit 18b controls the display unit 15 so that it displays the newly registered patient information of the current subject in place of the register finish information.

Next, the control unit 18 obtains the in-vivo images of the current subject captured by the capsule endoscope 2 in a state that the patient information of the current subject is newly registered in the storage unit 16 described above (step S205) and stores the in-vivo images of the current subject in the new folder created in the storage unit 16 at step S204 (step S206).

Specifically, after the patient information of the current subject is newly registered in the storage unit 16 and the display unit 15 displays it, the capsule endoscope 2 is introduced into the organs of the current subject. The capsule endoscope 2 captures in-vivo images while moving inside of the organs of the current subject and transmits a radio signal including the in-vivo images to the receiving device 10 worn on the current subject.
The receiving unit 11 of the receiving device 10 receives the radio signal and demodulates it to an image signal, and the image processing unit 13 creates the in-vivo images included in the image signal. The control unit 18 obtains the in-vivo images created by the image processing unit 13. In this case, the storage control unit 18c controls the storage unit 16 so that it causes the obtained in-vivo images (i.e. the in-vivo images of the current subject) to correspond to the patient information of the current subject and stores them. More specifically, the storage control unit 18c controls the storage unit 16 so that it stores the in-vivo images in a new folder that uses the patient information of the current subject as a folder name.

Thereafter, the control unit 18 determines whether or not the acquisition of the in-vivo images of the current subject have been finished (step S207). Specifically, when it is not instructed to finish to store the in-vivo images by operating the operating unit 14 described above as well as the predetermined time has not passed since the patient information is newly registered in the storage unit 16 at step S203 described above, the storage control unit 18c determines that the acquisition of the in-vivo images of the current subject have not been finished. Note that the predetermined time is the time corresponding to the time information previously set to the storage control unit 18c as described above. When the control unit 18 determines that the acquisition of the in-vivo images of the current subject have not been finished (step S207, No) as described above, the process returns to step S205 described above and repeats the processing procedure at step S205 and subsequent steps.

The storage control unit 18c repeats steps S205 to S207 described above until it determines that the acquisition of the in-vivo images of the current subject have been finished. In this case, the storage control unit 18c causes the storage unit 16 to store a series of in-vivo images, which is obtained from the image processing unit 13 during a period until it determines that the acquisition of the in-vivo images of the current subjects have been finished after the patient information of the current subject is newly registered in the storage unit 16 as a group of the in-vivo images of the current subject. More specifically, the storage control unit 18c controls the storage unit 16 so that it causes the series of the in-vivo images to correspond to the patient information newly registered at step S203 (the patient information of the current subject) and stores the series of the in-vivo images. Specifically, the storage control unit 18c controls the storage unit 16 so that it stores the series of the in-vivo images, i.e., the group of the in-vivo images of the current subject in the new folder that uses the newly registered patient information, which is the patient information of the current subject, as the folder name.

In contrast, when it is instructed to finish to store the in-vivo images by operating the operating unit 14 described above, or when a predetermined time has passed since the patient information is newly registered in the storage unit 16 at step S203, the storage control unit 18c determines that the acquisition of the in-vivo images of the current subject have been finished. When the storage control unit 18c determines that the acquisition of the in-vivo images of the current subject have been finished as described above (step S207: Yes), it finishes a storage processing of the in-vivo images to the new folder that uses the patient information of the current subject as the folder name (step S208). In this case, the storage control unit 18c finishes the control for the storage unit 16 to store the in-vivo images in the new folder as well as prohibits a storage process of an in-vivo image in the new folder.

Thereafter, the control unit 18 returns to step S201 described above and repeats the processing procedure at step S201 and subsequent steps. More specifically, the control unit 18 repeats the processing procedure at step S201 to S208 to the subjects to which the in-subject inspection described above is executed and causes the storage unit 16 to store therein the groups of the in-vivo images of the subjects by patient information. In this case, the storage control unit 18c causes the patient information registered in the storage unit 16 to correspond to the groups of the in-vivo images of the subjects specified by the patient information and causes the storage unit 16 to store therein the group of the in-vivo images of the subjects by patient information. Specifically, the storage control unit 18c controls the storage unit 16 so that it stores the groups of the in-vivo images, which are specified by the patient information that agree with folder names, in the folders $F_1$, ..., $F_n$ that use the patient information registered in the storage unit 16 as the folder names.

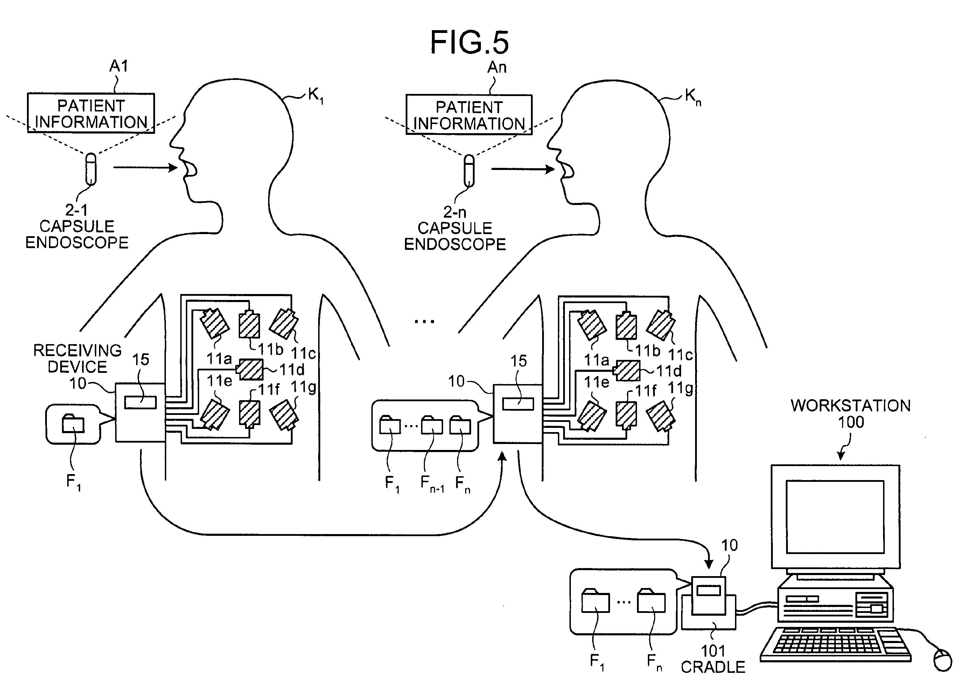

Next, an operation of the control unit 18 when it sequentially executes an in-subject inspection to subjects $K_1$, ..., $K_n$ will be specifically explained. FIG. 5 is a schematic view exemplifying a state that the in-subject inspection is executed to the subjects $K_1$, ..., $K_n$ using the in-vivo information acquiring device 1 according to the first embodiment. The user such as the doctor, the nurse, or the like repeats the job procedure at steps S101 to S104 described above to the subjects $K_1$, ..., $K_n$ and executes the in-subject inspection to them.

More specifically, as shown in FIG. 5, when the in-subject inspection is executed to the subjects $K_1$, ..., $K_n$, the receiving antennas 11a to 11g are dispersingly disposed on respective body surfaces of them, respectively. Further, optical information recording mediums A1, ..., An, on which the respective patient information of the subjects $K_1$, ... , $K_n$ are recorded, are prepared. Further, capsule endoscopes 2-1, ..., 2-n are allocated to the subjects $K_1$, ..., $K_n$, respectively. Note that each of the capsule endoscopes 2-1, ..., 2-n has the same configuration and the same function as those of the capsule endoscope 2 shown in FIG. 1.

The receiving device 10 is first worn on the subject $K_1$ as a first patient and connected the receiving antennas 11a to 11g dispersingly disposed on the body surface of the subject $K_1$. In this state, the receiving device 10 is started by being supplied with power from the power supply unit 19. In this case, the control unit 18 causes the display unit 15 to display the register request information described above.

When the register request information is displayed on the display unit 15, the user such as the doctor, the nurse, or the like registers the patient information of the subject $K_1$ in the receiving device 10. In this case, the capsule endoscope 2-1 captures a specific image including the patient information of the subject $K_1$ recorded to the optical information recording medium A1 before it is introduced into organs of the subject $K_1$ and wirelessly transmits the captured specific image to the outside. The receiving device 10 receives the specific image wirelessly transmitted by the capsule endoscope 2-1 through the receiving antennas 11a to 11g.

The control unit 18 obtains the patient information of the subject $K_1$ included in the received specific image and newly registers the obtained patient information in the storage unit 16. Then, the control unit 18 newly creates a folder $F_1$ that uses the newly registered patient information as a folder name in the storage unit 16. Next, the control unit 18 controls the display unit 15 so that it displays register finish information for a predetermined time in place of the register request information described above and controls the display unit 15 so that it displays the patient information of the subject $K_1$ in place of the register finish information after the predetermined time passes.

In this case, the display unit 15 displays the register request information described above for a predetermined time in place of the register finish information and displays the patient information of the subject $K_1$ (new patient information) after the predetermined time passes in place of the register finish information. The user can understand that the patient information of the subject $K_1$ is registered in the receiving device 10 by visually recognizing the display information of the display unit 15 (register finish information, new patient information).

Thereafter, the capsule endoscope 2-1 is swallowed from a mouth of the subject $K_1$ and introduced into organs of the subject $K_1$. In this case, the capsule endoscope 2-1 sequentially captures in-vivo images at predetermined intervals (intervals of, for example, 0.25 second) while moving inside of the organs of the subject $K_1$ and wirelessly transmits the obtained in-vivo images to the outside sequentially. The receiving device 10 sequentially receives the in-vivo images from the capsule endoscope 2-1 in the organs through the receiving antennas 11a to 11g.

The control unit 18 obtains a series of the in-vivo images sequentially captured by the capsule endoscope 2-1 inside of the organs during a period until a predetermined time (the time corresponding to the previously set time information) passes after the patient information of the subject $K_1$ is newly registered in the storage unit 16. The control unit 18 controls the storage unit 16 so that it stores the series of the in-vivo images, i.e., a group of the in-vivo images of the subject $K_1$ in the folder $F_1$. As a result, the control unit 18 causes the patient information of the subject $K_1$ as the folder name of the folder $F_1$ to correspond to the group of the in-vivo images of the subject $K_1$.

After the predetermined time passes, the control unit 18 finishes a control of storing the in-vivo images in the folder $F_1$ to the storage unit 16 as well as prohibits a storage process of the in-vivo images to the folder $F_1$. As described above, the control unit 18 has obtained the group of the in-vivo images of the subject $K_1$. Thereafter, the control unit 18 causes the display unit 15 to display the register request information described above using the fact, as a trigger, that the predetermined time has passed. The user understands that the group of the in-vivo images of the subject $K_1$ has been obtained by visually recognizing the display information of the display unit 15 and removes the receiving device 10 from the subject $K_1$. At the time, the receiving device 10 holds the group of the in-vivo images of the subject $K_1$ in

the folder $F_1$ of the storage unit 16.

Thereafter, an in-subject inspection for obtaining a group of in-vivo images of an n-th subject $K_n$ (n = 2, 3, ...) is executed by the same job procedure (the job procedure at steps S101 to S104 described above) as that of the first subject $K_1$ described above.

More specifically, as shown in FIG. 5, the capsule endoscope 2-n captures a specific image including the patient information of the subject $K_n$ recorded on the optical information recording medium An before it is introduced into organs of the subject $K_n$ and wirelessly transmits the captured specific image to the outside. The receiving device 10 worn on the subject $K_n$ receives the specific image wirelessly transmitted by the capsule endoscope 2-n through the receiving antennas 11a to 11g.

The control unit 18 of the receiving device 10 newly registers the patient information of the subject $K_n$ to the storage unit 16 by executing the same processing procedure as that of the subject $K_1$ described above and newly creates a folder $F_n$ in the storage unit 16 using the newly registered patient information as a folder name. In this case, the display unit 15 of the receiving device 10 sequentially displays register request information and register finish information likewise the case of the subject $K_1$ described above and displays the patient information of the subject $K_n$ in place of the register finish information after a predetermined time passes.

Thereafter, the capsule endoscope 2-n is swallowed from a mouth of the subject $K_n$ and introduced into organs of the subject $K_n$. In this case, the capsule endoscope 2-n sequentially captures in-vivo images at predetermined intervals (intervals of, for example, 0.25 second) while moving inside of the organs of the subject $K_n$ and wirelessly transmits the obtained in-vivo images to the outside sequentially. The receiving device 10 sequentially receives the in-vivo images from the capsule endoscope 2-n in the organs through the receiving antennas 11a to 11g.

In this case, the control unit 18 of the receiving device 10 controls the storage unit 16 so that it stores a series of the in-vivo images, which is obtained during a period until a predetermined time passes after the patient information of the subject $K_n$ is newly registered in the storage unit 16, i.e., a series of in-vivo images sequentially captured by the capsule endoscope 2-n in the folder $F_n$ likewise the subject $K_1$ described above.

The control unit 18 prohibits the folders $F_1$, ..., $F_{n-1}$ already created in the storage unit 16 of the receiving device 10 to execute a storage process for newly storing an in-vivo image. More specifically, a folder, which can newly store the in-vivo images among the folders $F_1$, ..., $F_{n-1}$, $F_n$ in the storage unit 16, is only the new folder $F_n$. The control unit 18 executes a control for storing a group of the in-vivo images of the subject $K_n$ in the new folder $F_n$ to thereby cause the patient information of the subject $K_n$ as the folder name of the folder $F_n$ to correspond to the group of the in-vivo images of the subject $K_n$.

After a predetermined time passes likewise the case of the subject $K_1$ described above, the control unit 18 finishes a control for storing the in-vivo images in the folder $F_n$ to the storage unit 16 as well as prohibits a storage process of the in-vivo images to the folder $F_n$. As described above, the control unit 18 has obtained the group of the in-vivo images of the subject $K_n$. At the time, the receiving device 10 displays register request information on the display unit 15 likewise the case of the subject $K_1$ described above as well as holds the groups of the in-vivo images of the subjects $K_1$, ..., $K_n$ in the folders $F_1$, ..., $F_n$ of the storage unit 16, respectively. In this case, the folders $F_1$, ..., $F_n$ store the groups of the in-vivo images of the subjects $K_1$, ..., $K_n$ specified by the patient information as the folder names, respectively.

Thereafter, the user understands that the acquisitions of the groups of the in-vivo images of the subjects $K_1$, ..., $K_n$ have been finished by visually recognizing the register request information displayed on the display unit 15 of the receiving device 10 and removes the receiving device 10 from the subject $K_n$. After the receiving device 10, which holds the groups of the in-vivo images of the subjects $K_1$, ..., $K_n$ for each piece of the patient information, is removed from the subject $K_n$, it is placed on a cradle 101 of a workstation 100.

The workstation 100 has the cradle 101 connected through a cable and the like and an image display function for displaying the groups of the in-vivo images captured by the capsule endoscope 2. The workstation 100 is connected to the output unit 17 of the receiving device 10 through the cradle 101. The user causes the workstation to collectively capture the groups of the in-vivo images of the subjects stored in the receiving device 10 by a download job executed once.

In the download job executed once, the workstation 100 captures the groups of the in-vivo images of the subjects $K_1$, ..., $K_n$ from the storage unit 16 of the receiving device 10 through the cradle 101, and the output unit 17, and the like at a time. In this case, the workstation 100 captures the groups of the in-vivo images of the subjects $K_1$, ..., $K_n$ together with the folders $F_1$, ..., $F_n$ described above. The workstation causes the groups of the in-vivo images of the subjects $K_1$, ..., $K_n$ captured from the receiving device 10 to correspond to the patient information of the respective subjects and holds and manages the groups of the in-vivo images of the subjects $K_1$, ..., $K_n$ for each piece of the patient information.

Thereafter, the user operates the workstation 100 which has captured the groups of the in-vivo images of the subjects $K_1$, ..., $K_n$, and displays the group of the in-vivo images of a desired subject of the subjects $K_1$, ..., $K_n$ on the display of the workstation 100. Then, the user diagnoses the desired subject by observing the group of the in-vivo images displayed on the workstation 100. The user achieves the in-subject inspection to the subjects $K_1$, ..., $K_n$ as described

above.

Note that the storage unit 16 of the receiving device 10 has a storage capacity necessary to store the groups of the in-vivo images of the subjects by patient information as described above. There is assumed a case, for example, that the imaging unit 4 of the capsule endoscope 2 captures in-vivo images at intervals of 0.25 second (i.e., the imaging unit 4 has a capturing rate of 4 frames/second), a series of in-vivo images, which is captured by the capsule endoscope 2 until 30 minutes passes after the patient information of a subject is newly registered in the storage unit 16, is stored in a new folder (i.e., the time corresponding to the time information previously set to the storage control unit 18c is 30 minutes), a data amount of in-vivo images of one frame is about 30 K bytes, and an in-subject inspection is executed to ten subjects. In this case, a storage capacity M necessary to the storage unit 16 is calculated by the following expression (1) and about 2.2 gigabytes.

$$\text{Storage capacity M} = 4\ [\text{fps}] \times 3600\ [\text{sec/h}] \times 0.5\ [\text{h}]$$
$$\times\ 30\ [\text{kBytes}] \times 10\ [\text{persons}] \qquad\qquad (1)$$

Further, the storage unit 16 can store therein the group of the in-vivo images of a desired portion of the subject by patient information by that the time information corresponding to a moving time until the capsule endoscope 2 reaches the desired portion in the subject is previously set to the storage control unit 18c. Specifically, when the time information corresponding to about 0.5 to 1 hour is previously set to the storage control unit 18c, the storage unit 16 can store therein a group of in-vivo images from an oral cavity to a stomach of a subject by patient information, when the time information corresponding to about 2 to 8 hours is previously set to the storage control unit 18c, it can store a group of in-vivo images from the oral cavity to a small intestine of the subject by patient information, and when the time information corresponding to more than 8 hours is previously set to the storage control unit 18c, it can store a group of in-vivo images of all the regions of an alimentary canal from the oral cavity to an anus of the subject by patient information.

As explained above, according to the first embodiment, the specific information for specifying a subject is obtained; the specific information is registered in the storage unit for storing a group of in-vivo images captured by the capsule endoscope introduced into organs of the subject; and the registered specific information is caused to correspond to a group of in-vivo images of the subject specified by the specific information so as to store groups of in-vivo images of the subjects in the storage unit by the specific information. Therefore, the storage unit can store therein the groups of the in-vivo images of the subjects while keeping a state that the in-vivo images can be identified for each piece of the specific information (for example, for each subject) so that the groups of the in-vivo images of the subjects stored in the storage unit by specific information can be collectively downloaded to an external device such as the workstation and the like. As a result, since repetition of a series of trouble jobs for causing the external device to capture a group of in-vivo images of a subject each time it is stored in the storage unit can be omitted, there can be realized an in-vivo information acquiring device which can smoothly execute an in-subject inspection for acquiring a group of in-vivo images of a subject to a plurality of subjects.

Further, the in-vivo information acquiring device is configured such that a specific image including the specific information of a subject is captured by the imaging unit of the capsule endoscope introduced into organs of the subject to thereby obtain the specific information of the subject. Therefore, since the specific information of the subject can be obtained just before the capsule endoscope is introduced into the organs of the subject, the specific information of the subject can be securely registered in the storage unit in which a group of in-vivo images of the subject is stored. As a result, the specific information of the subject registered in the storage unit can be caused to securely correspond to the group of the in-vivo images of the subject.

Further, the imaging unit of the capsule endoscope is cofigured to capture a specific image including the specific information of a subject and a group of in-vivo images of the subject. Therefore, the capsule endoscope introduced into organs of a subject can be made compact and can be suppressed from being burdened by the subject when it is introduced into the organs.

Second Embodiment

Next, an second embodiment of the invention will be explained. The first embodiment described above obtains the patient information of a subject by capturing a specific image including patient information specifying the subject. However, the second embodiment is arranged such that a receiving means is contained in a capsule endoscope introduced into organs of a subject and receives the patient information of the subject by executing a wireless communication by the receiving means so that the patient information of the subject can be obtained.

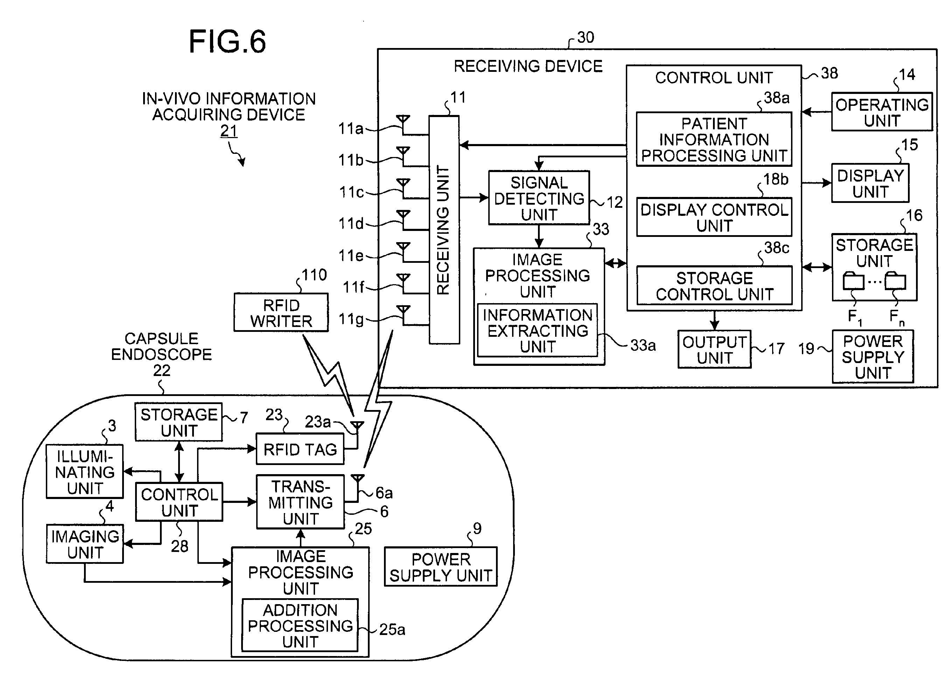

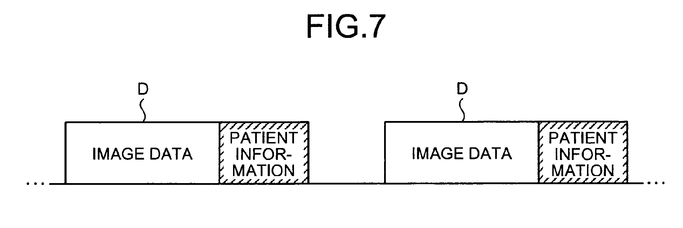

FIG. 6 is a block diagram schematically showing a configuration example of an in-vivo information acquiring device according to the second embodiment of the invention. FIG. 7 is a schematic view exemplifying an image signal including image data of an in-vivo image and the patient information of a subject. As shown in FIG. 6, an in-vivo information acquiring device 21 according to the second embodiment has a capsule endoscope 22 in place of the capsule endoscope 2 of the in-vivo image acquiring device 1 according to the first embodiment described above and a receiving device 30 in place of the receiving device 10. Note that an RFID writer 110 is an external communication device which can input the patient information of a subject and has a function for writing the patient information of a subject in the capsule endoscope 22 by means of wirelessly communicating therewith. The other cofigurations are the same as those of the first embodiment, and the same reference numerals are given to the same components. A configuration of the capsule endoscope 22 will be explained first, and next a configuration of the receiving device 30 will be explained below referring to FIGS. 6 and 7.

The capsule endoscope 22 obtains the patient information of a subject by means of wirelessly communicating with the external RFID writer 110 in place of capturing a specific image as in the capsule endoscope 2 of the in-vivo information acquiring device 1 according to the first embodiment described above. Further, the capsule endoscope 22 adds the patient information of the subject obtained as described above to image signals and sequentially transmits radio signals obtained by modulating the image signals (that is, image signals including in-vivo images and patient information) to the receiving device 30. The other functions of the capsule endoscope 22 are the same as those of the capsule endoscope 2 of the first embodiment described above.

The capsule endoscope 22 has an image processing unit 25 in place of the image processing unit 5 of the capsule endoscope 2 of the in-vivo information acquiring device 1 according to the first embodiment described above, a control unit 28 in place of the control unit 8, and further an RFID tag 23 which is a receiving means of patient information. Further, the imaging unit 4 of the capsule endoscope 22 does not capture the specific image described above but sequentially captures in-vivo images of a subject at predetermined intervals. The other configurations of the capsule endoscope 22 are the same as those of the capsule endoscope 2 of the first embodiment described above, and the same reference numerals are given to the same components.

The RFID tag 23 acts as a receiving means for receiving the patient information of a subject by means of executing a wireless communication. Specifically, the RFID tag 23 includes a coil, a condenser, and the like and has an antenna 23a formed by the coil. The RFID tag 23 wirelessly communicates with the external RFID writer 110, which can be input with and holds the patient information of a subject, and receives the patient information of a subject from the RFID writer 110 through the antenna 23a. As described above, the RFID tag 23 obtains the patient information of the subject. Thereafter, the RFID tag 23 transmits the obtained patient information to the control unit 28.

The image processing unit 25 creates an image signal including an in-vivo image and the patient information of a subject. Specifically, the image processing unit 25 obtains image data of a subject image (for example, an in-vivo image) captured by the imaging unit 4 therefrom and obtains the patient information of a subject received by the RFID tag 23 from the control unit 28. In this case, the image processing unit 25 obtains image data of the same subject as the image data of a subject (i.e. the image data of the subject into organs of which the capsule endoscope 22 is introduced) obtained from the imaging unit 4 from the control unit 28. Thereafter, the image processing unit 25 creates image signals including the image data, patient information, and the like and sequentially transmits the created image signals to the transmitting unit 6 each time when the image processing unit 25 creates the image signals.

The image processing unit 25 has an addition processing unit 25a. The addition processing unit 25a adds patient information to an image signal including image data of an in-vivo image. Specifically, when the imaging unit 4 captures an in-vivo image, the image processing unit 25 creates an image signal including the in-vivo image, inherent identification parameters such as a capsule ID and the like for specifying the capsule endoscope 22, various image processing parameters, and the like likewise the first embodiment described above. The addition processing unit 25a adds the patient information obtained from the control unit 28 (i.e., the patient information of a subject received by the RFID tag 23 from the RFID writer 110) to the image signal including the in-vivo image and the like. In this case, the addition processing unit 25a adds the patient information to image data D of each of respective in-vivo images sequentially obtained from the imaging unit 4 as shown in, for example, FIG. 7. With this operation, the addition processing unit 25a causes the image data D of each in-vivo image to correspond to the patient information.

Note that the image signals including in-vivo images, the inherent identification parameters such as the capsule ID and the like, the various image processing parameters, the patient information, and the like are sequentially transmitted to the transmitting unit 6 as described above. In this case, each time when the transmitting unit 6 obtains the image signals from the image processing unit 25, it creates radio signals by modulating the image signals. The transmitting unit 6 sequentially transmits the radio signals to the external receiving device 30 through a transmitting antenna 6a.

The control unit 28 controls the respective units (an illuminating unit 3, the imaging unit 4, the image processing unit 25, the transmitting unit 6, and the storage unit 7) of the capsule endoscope 22 and controls signals input and output between the respective units. In this case, the control unit 28 obtains the patient information of the subject from the RFID tag 23 and controls the storage unit 7 so that it stores the obtained patient information. Further, the control unit 28 transmits the patient information stored in the storage unit 7 to the image processing unit 25 and controls the image processing unit 25 so that it creates an image signal including the transmitted patient information and an in-vivo image including an in-vivo image and the like of a subject specified by the patient information. The other functions of the control unit 28 are the same as those of the control unit 8 of the capsule endoscope 2 of the first embodiment described above.

Next, a configuration of the receiving device 30 will be explained. The receiving device 30 receives an image signal including patient information and an in-vivo image of a subject (specifically, a radio signal obtained by modulating the image signal) from the capsule endoscope 22 in place of receiving a specific image including the patient information of a subject and obtains the patient information included in the received image signal together with the in-vivo image. Further, the receiving device 30 registers the obtained patient information in the storage unit 16, causes the registered patient information to correspond to an in-vivo image of a subject specified by the patient information, and stores groups of in-vivo images of subjects in the storage unit 16 by patient information. In this case, the receiving device 30 compares the patient information, which is registered in the storage unit 16, with the patient information, which is newly obtained together with the in-vivo image and causes the in-vivo image, which is obtained together with patient information that agrees with the registered patient information to correspond to the registered patient information. The other functions of the receiving device 30 are the same as those of the receiving device 10 of the first embodiment described above.

The receiving device 30 has an image processing unit 33 in place of the image processing unit 13 of the receiving device 10 of the in-vivo information acquiring device 1 according to the first embodiment described above and has a control unit 38 in place of the control unit 18. The other configurations of the receiving device 30 are same as those of the receiving device 10 of the first embodiment described above, and the same reference numerals are given to the same components.

The image processing unit 33 extracts the patient information of a subject included in an image signal wirelessly transmitted by the capsule endoscope 22 as well as creates an in-vivo image included in the image signal. Specifically, the image processing unit 33 sequentially obtains the image signals per frame detected by the signal detecting unit 12. Each of the image signals per frame includes image data of an in-vivo image of one frame, the inherent identification parameters such as the capsule ID and the like of the capsule endoscope 22, the image processing parameters such as white balance and the like, patient information, and the like. The image processing unit 33 creates the in-vivo image of one frame using image data of the in-vivo image of one frame and the various image processing parameters.

Further, the image processing unit 33 has an information extracting unit 33a for extracting patient information. The information extracting unit 33a extracts the patient information of a subject included in the image signal per frame. The patient information extracted by the information extracting unit 33a is the patient information of the subject obtained by capturing the in-vivo image included in the image signal per frame as described above. Each time when the image processing unit 33 obtains the in-vivo images and the patient informations of the one frame and the capsule ID based on the image signal of the frame unit, it sequentially transmits the in-vivo images and the patient information of the one frame and the capsule ID to the control unit 38.

The control unit 38 controls the respective units (the receiving unit 11, the signal detecting unit 12, the image processing unit 33, the operating unit 14, the display unit 15, the storage unit 16, and the output unit 17) of the receiving device 30 and controls signals input and output between the respective units. In this case, the control unit 38 registers the patient information of the subject received by the RFID tag 23 of the capsule endoscope 22 described above in the storage unit 16 and compares the registered patient information with patient information newly obtained together with an in-vivo image. The control unit 38 controls the storage unit 16 so that it stores groups of in-vivo images of subjects by registered patient information based on a result of the comparison process of the patient information. The other functions of the control unit 38 are the same as those of the control unit 18 of the receiving device 10 of the first embodiment described above.

The control unit 38 has a patient information processing unit 38a in place of the patient information processing unit 18a of the control unit 18 of the receiving device 10 described above, a storage control unit 38c in place of the storage control unit 18c, and the display control unit 18b described above.

The patient information processing unit 38a sequentially obtains the patient information of subjects for each in-vivo image which are transmitted together with the in-vivo images of one frame by the image processing unit 33. Each time the patient information processing unit 38a newly obtains the patient information of a subject, it searches the newly obtained patient information from the patient information already registered in the storage unit 16. The patient information processing unit 38a determines whether or not the newly obtained patient information has been registered in the storage unit 16 based on a result of search of the patient information. When the patient information processing unit 38a determines that the newly obtained patient information has not been registered in the storage unit 16 yet, it newly registers the newly obtained patient information in the storage unit 16. Each time the patient information processing unit 38a newly registers the patient information in the storage unit 16 as described above, it creates a folder $F_n$ (n = 1, 2, ...) in the storage unit 16 using the new patient information as a folder name. The patient information processing unit 38a creates a plurality of folders $F_1$, ..., $F_n$ in each piece of the patient information in the storage unit 16 as described above. When the patient information processing unit 38a determines that the newly obtained patient information has been already registered in the storage unit 16, it notifies the storage control unit 38c of the newly obtained patient information.

The storage control unit 38c controls the storage unit 16 so that it stores the groups of the in-vivo images of subjects by the patient information registered in the storage unit 16 by the patient information processing unit 18a described above. Specifically, the storage control unit 38c compares the patient information registered in the storage unit 16 by the patient information processing unit 38a with the patient information newly obtained from the image processing unit 33 together with the in-vivo image. Note that the patient information, which is newly obtained by the storage control unit 38c together with the in-vivo image, is the patient information the register of which in the storage unit 16 is determined by the patient information processing unit 38a described above. The storage control unit 38c causes the in-vivo image, which is newly obtained together with the patient information that agrees with the patient information already registered by the patient information processing unit 38a in the storage unit 16, to correspond to the registered patient information bases on a result of the comparison process of the patient information. In this case, the storage control unit 38c controls the storage unit 16 so that it stores the newly obtained in-vivo image together with the patient information in the folder $F_n$ (n = 1, 2, ...) using the patient information that agrees with the newly obtained patient information as a folder name.

Further, the storage control unit 38c determines, for each piece of the patient information, whether or not the acquisition of the groups of the in-vivo images of the subjects to be stored in the storage unit 16 have been finished likewise the storage control unit 18c of the receiving device 10 of the first embodiment described above. The storage control unit 38c controls the storage unit 16 so that it stores a series of in-vivo images (i.e., a group of in-vivo images captured by the capsule endoscope 22 inside of organs of a current subject), which is sequentially obtained from the image processing unit 33 during a period from a time at which the patient information processing unit 38a newly registers the patient information of the current subject in the storage unit 16 to a time at which the patient information processing unit 38a determines that the acquisition of a group of in-vivo images of the current subject has been finished, in a folder that uses the patient information of the current subject as a folder name. Further, the storage control unit 38c stores a capsule ID and the like of the capsule endoscope 22 together with the patient information and the in-vivo images of the current subject in the folder. As described above, the storage control unit 38c causes the patient information of the current subject, the group of the in-vivo images of the current subject, and the capsule ID of the capsule endoscope 22 introduced into the organs of the current subject to correspond to each other.

The storage control unit 38c repeats the same process and the same control as to a plurality of subjects and causes the storage unit 16 to store the groups of the in-vivo images of the subjects in each piece of the patient information. In this case, a group of in-vivo images of a subject specified by the patient information as the folder name, the capsule ID, and the like are stored in the folder $F_n$ (n = 1, 2, ...) in the storage unit 16.

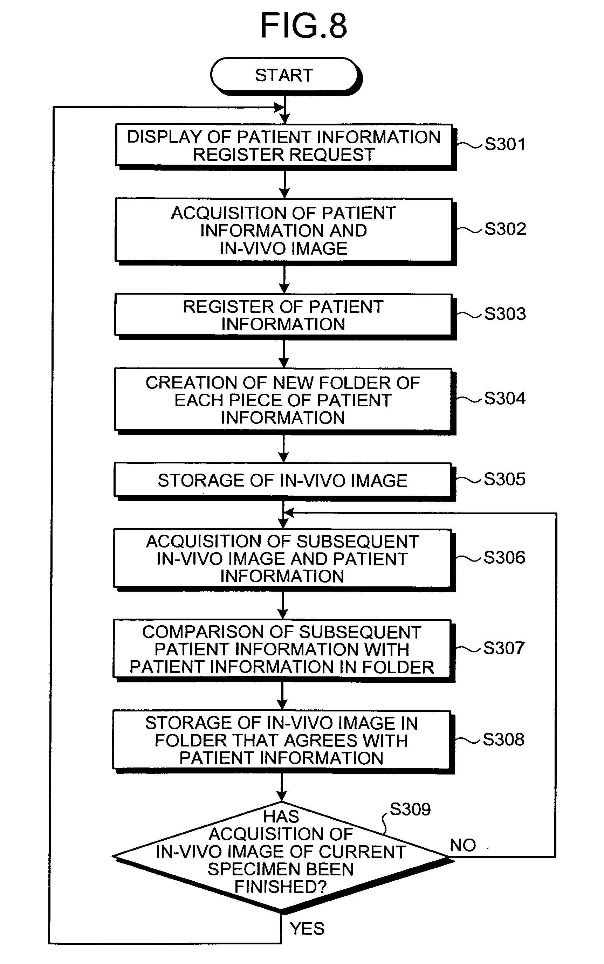

Next, an operation of the control unit 38 of the receiving device 30 for storing groups of in-vivo images of subjects by the patient information when the in-subject inspection described above is executed to the subjects will be explained. FIG. 8 is a flowchart exemplifying a processing procedure of the control unit 38 of the receiving device 30 of the second embodiment.

As shown in FIG. 8, first, the control unit 38 causes the display unit 15 to display a register request of the patient information of a subject (step S301). In this case, the control unit 38 causes the display unit 15 to display register request information likewise step S201 described above.

Next, the control unit 38 obtains the patient information and the in-vivo image of the current subject on which the receiving device 30 is worn to execute the in-subject inspection (step S302). Specifically, the RFID tag 23 of the capsule endoscope 22 receives the patient information of the current subject from the RFID writer 110 according to the register request information displayed on the display unit 15 at step S301. The capsule endoscope 22, which obtains the patient information of the current subject by the RFID tag 23, is introduced into the organs of the current subject and captures an in-vivo image of the current subject. The capsule endoscope 22 transmits a radio signal including the in-vivo image and the patient information of the current subject to the receiving device 30 worn on the current subject. The receiving unit 11 of the receiving device 30 receives the radio signal and demodulates it to an image signal, and the image processing unit 33 obtains the in-vivo image and the patient information of the current subject included in the image signal. The control unit 38 obtains the in-vivo image and the patient information of the current subject from the image processing unit 33.

Thereafter, the control unit 38 registers the patient information of the current subject newly obtained at step S302 (step S303) and newly creates a folder of each piece of the patient information using the registered patient information (step S304). In this case, the patient information processing unit 38a searches the storage unit 16 for the patient information described above and determines that the patient information of the current subject has not been registered in the storage unit 16 yet based on a result of search for the patient information. The patient information processing unit 38a writes the patient information of the current subject in the storage unit 16 to thereby newly register it in the storage unit 16. Then, the patient information processing unit 38a newly creates a folder that uses the newly registered patient information (i.e., the patient information of the current subject) as a folder name in the storage unit 16.

Next, the control unit 38 stores the in-vivo image of the current subject obtained together with the patient information at step S302 in the folder of the storage unit 16 (step S305). In this case, the storage control unit 38c controls the storage unit 16 so that it causes the in-vivo image of the current subject to correspond to the patient information of the current subject and stores the in-vivo image of the current subject. More specifically, the storage control unit 38c controls the storage unit 16 so that it stores the in-vivo image of the current subject in the folder (the folder that uses the patient information of the current subject as the folder name) newly created at step S304.

Note that, when the patient information processing unit 38a newly registers the patient information in the storage unit 16, the display control unit 18b controls the display unit 15 so that it sequentially displays register finish information and the patient information of the current subject likewise the first embodiment described above.

Thereafter, the control unit 38 obtains an in-vivo image (a subsequent in-vivo image of the current subject) continuously captured by the capsule endoscope 22 in the organs of the current subject and the patient information of the current subject in the state that the patient information of the current subject is newly registered in the storage unit 16 (step S306). In this case, the control unit 38 obtains the patient information of the current subject together with the subsequent in-vivo image from the image processing unit 33 again. The patient information of the current subject, which is obtained again by the control unit 38 together with the subsequent in-vivo image is wirelessly transmitted from the capsule endoscope 22 together with the subsequent in-vivo image and is subsequent patient information subsequent to the patient information of the current subject previously obtained by the control unit 38.

When the control unit 38 obtains the subsequent patient information and the subsequent in-vivo image of the current subject, it compares the subsequent patient information with the patient information of the folder in the storage unit 16 (step S307) and stores the subsequent in-vivo image of the current subject in the folder with which the patient information agrees (step S308). In this case, the storage control unit 38c compares the subsequent patient information, which is obtained together with the subsequent in-vivo image, with the patient information already registered in the storage unit 16 (i.e., the patient information registered as the folder name of the folder in the storage unit 16). The storage control unit 38c specifies a folder that uses patient information which agrees with the subsequent patient information as a folder name based on a result of comparison of the patient information. Note that the folder specified by the storage control unit 38c is a folder that uses the patient information of the current subject as a folder name. The storage control unit 38c controls the storage unit 16 so that it stores of the subsequent in-vivo images obtained at step S306 in the specified folder. With this operation, the storage control unit 38c causes the patient information of the current subject, which is the folder name of the specified folder (i.e., the folder newly created at step S304) to correspond to the subsequent in-vivo image.

Thereafter, the control unit 38 determines whether or not the acquisition of the in-vivo images of the current subject have been finished likewise step S207 described above (step S309). When the control unit 38 determines that the acquisition of the in-vivo images of the current subject have not been finished (step S309: No), the process returns to step S306 described above and repeats the processing procedure at step S306 and subsequent steps. That is, the storage control unit 38c repeats steps S306 to S309 described above until it determines that the acquisition of the in-vivo images of the current subject have been finished.

The storage control unit 38c causes the storage unit 16 to store a series of in-vivo images obtained from the image processing unit 33 during a period until it determines that the acquisition of the in-vivo images of the current subjects have been finished since the patient information of the current subject is newly registered in the storage unit 16 as a group of the in-vivo images of the current subject. More specifically, the storage control unit 38c controls the storage unit 16 so that it causes the series of the in-vivo images to correspond to the patient information of the current subject newly registered at step S303 and to store the series of the in-vivo images. In this case, the storage control unit 38c controls the storage unit 16 so that it stores the series of the in-vivo images,i.e., the group of the in-vivo images of the current subject in the folder that uses the patient information of the current subject as the folder name.

In contrast, when the control unit 38 determines that the acquisition of the in-vivo images of the current subject have been finished at step S309 (step S309: Yes), it returns to step S301 described above and repeats the processing procedures at step S301 and subsequent steps. More specifically, the control unit 38 repeats the processing procedure at step S301 to S309 to subjects to which the in-subject inspection described above is executed and causes the storage unit 16 to store groups of in-vivo images of the subjects by the patient information.

In this case, the storage control unit 38c causes the patient information registered in the storage unit 16 to correspond to groups of in-vivo images of subjects specified by the patient information and causes the storage unit 16 to store the groups of the in-vivo images of the subjects by the patient information. Specifically, the storage control unit 38c controls the storage unit 16 so that it stores the groups of the in-vivo images, which are specified by the patient information that agree with folder names, in the folders $F_1$, ..., $F_n$ that use the patient information registered in the storage unit 16 as the folder names.

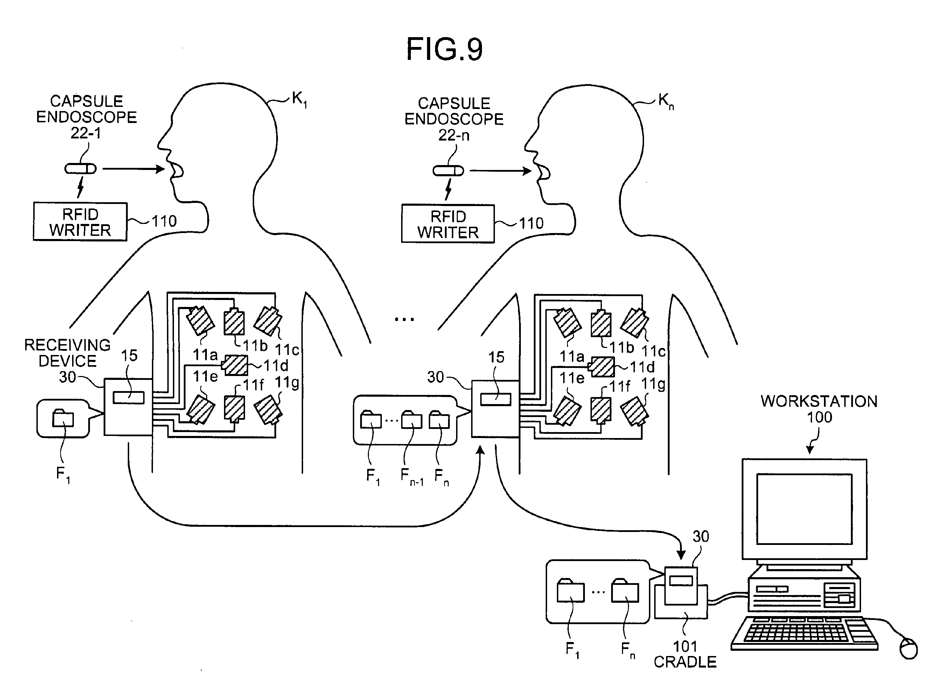

Next, an operation of the control unit 38 when it sequentially executes the in-subject inspection to the subjects $K_1$, ..., $K_n$ will be specifically explained. FIG. 9 is a schematic view exemplifying a state that the in-subject inspection is executed to the subjects $K_1$, ..., $K_n$ using the in-vivo information acquiring device 21 according to the second embodiment.

When the in-vivo information acquiring device 21 according to the second embodiment is used, a user such as a doctor, a nurse, or the like repeats a job procedure approximately the same as that of steps S101 to S104 shown in FIG. 3 to the subjects $K_1$, ..., $K_n$, and then executes the job procedure at steps S105 and S106 shown in FIG. 3 to thereby achieve the in-subject inspection to the subjects $K_1$, ..., $K_n$. A difference between the first and the second embodiments in the in-subject inspection to subjects will be explained below referring to FIG. 9.

Further, a plurality of capsule endoscopes 22-1, ..., 22-n are allocated to the subjects $K_1$, ..., $K_n$, respectively to which the in-subject inspection is executed. Note that each of the capsule endoscopes 22-1, ..., 22-n has the same configuration as and the same function as those of the capsule endoscope 22 shown in FIG. 6.

At steps S101 and S102 described above, when register request information is displayed on the display unit 15 of the receiving device 30 worn on the subject $K_1$, the user such as the doctor, the nurse, or the like registers the patient information of the subject $K_1$ in the receiving device 30 using the RFID writer 110. In this case, before the capsule endoscope 22-1 is introduced into organs of the subject $K_1$, it wirelessly communicates with the RFID writer 110 and receives the patient information of the subject $K_1$ therefrom. The user introduces the capsule endoscope 22-1, which obtains the patient information of the subject $K_1$ as described above into the organs of the subject $K_1$. The capsule endoscope 22-1 captures an in-vivo image of the subject $K_1$ while moving in the organs and wirelessly transmits the patient information of the subject $K_1$ together with the captured in-vivo image to the outside.

The receiving device 30 receives the in-vivo image and the patient information of the subject $K_1$ wirelessly transmitted by the capsule endoscope 22-1 in the organs through the receiving antennas 11a to 11g. In this case, the control unit 38 determines that the patient information of the subject $K_1$ obtained together with the in-vivo image is not yet registered in the storage unit 16 and newly registers the patient information of the subject $K_1$ in the storage unit 16 based on the determination. Then, the control unit 38 newly creates the folder $F_1$ using the newly registered patient information of the patient $K_1$ as a folder name in the storage unit 16. Subsequently, the control unit 38 controls the storage unit 16 so that it stores the in-vivo image of the subject $K_1$ obtained together with the patient information in the folder $F_1$.

Thereafter, the capsule endoscope 22-1 sequentially captures in-vivo images at predetermined intervals (intervals of, for example, 0.25 second) while moving in the organs of the subject $K_1$ at step S103 described above and wirelessly transmits an image signal including the patient information and the in-vivo images of the subject $K_1$ sequentially. The receiving device 30 sequentially receives the patient information and the in-vivo images of the subject $K_1$ from the capsule endoscope 22-1 in the organs through the receiving antennas 11a to 11g.

In this case, the control unit 38 executes a comparison process of the patient information as described above and specifies the folder $F_1$, which uses the patient information of the subject $K_1$ as the folder name, as a folder for storing a group of the in-vivo images of the subject $K_1$. The control unit 38 controls the storage unit 16 so that it stores a series of the in-vivo images in the folder $F_1$, which is obtained together with the patient information of the subject $K_1$ until a predetermined time (the time corresponding to the previously set time information) has passed since the patient information of the subject $K_1$ is newly registered in the storage unit 16. With this operation, the control unit 38 causes the patient information of the subject $K_1$, which is the folder name of the folder $F_1$, to the group of the in-vivo images of the subject $K_1$.

After the predetermined time passes, the control unit 38 determines that the acquisitio of the group of the in-vivo images of the subject $K_1$ has been finished and causes the display unit 15 to display the register request information described above. Thereafter, the in-subject inspection is executed to the n-th subject $K_n$ (n = 2, 3, ...) by approximately the same as that of the first subject $K_1$.

In this case, the control unit 38 controls the storage unit 16 so that it executes the comparing process for comparing the respective folder names (i.e. the patient information of the subjects) of the folders $F_1$, ..., $F_{n-1}$, $F_n$ created in the storage unit 16 of the receiving device 10 with the patient information described above, specifies a folder for storing the in-vivo images of the current subject, and stores the group of the in-vivo images of the current subject in the specified folder. With this operation, the control unit 38 causes the patient information of the subject $K_n$ as the folder name of the folder $F_n$ to correspond to the group of the in-vivo images of the subject $K_n$.

As described above, the control unit 38 causes the respective patient information of the subjects $K_1$, ..., $K_n$ as the respective folder names of the folders $F_1$, ..., $F_n$ to correspond to the groups of the in-vivo images of the subjects $K_1$, ..., $K_n$. The receiving device 30 holds the groups of the in-vivo images of the subjects $K_1$, ..., $K_n$ in the folders $F_1$, ..., $F_n$ of the storage unit 16 based on control of the control unit 38.

After the receiving device 30 is connected to the workstation 100 through the cradle 101 and the like likewise the first embodiment described above, the groups of the in-vivo images of the subjects $K_1$, ..., $K_n$ stored in the receiving device 30 are collectively captured by the workstation 100 by a download job executed once.

As explained above, according to the second embodiment of the invention, the specific information of a subject into organs of which the capsule endoscope is introduced is written in the storage unit of the capsule endoscope for capturing an in-vivo image of the subject; the capsule endoscope wirelessly transmits the specific information and the in-vivo image of the subject together; the specific information wirelessly transmitted together with the in-vivo image is registered in the storage unit for storing a group of in-vivo images captured by the capsule endoscope; and the registered specific information is caused to correspond to the group of the in-vivo images of the subject specified by the specific information so as to store groups of in-vivo images of a plurality of subjects in the storage unit by the specific information. Therefore, since a specific information registered in the storage unit can be compared with the subsequent specific information of a subsequent in-vivo image, the same operation/working effect as that of the first embodiment described above can be obtained as well as the specific information registered in the storage unit can be more securely caused to correspond to a group of in-vivo images of the subject specified by the specific information.

Third Embodiment

Next, an third embodiment of the invention will be explained. In the first embodiment described above, the patient information of a subject is obtained by capturing a specific image including the patient information for specifying the subject. However, the third embodiment is configured such that the patient information of a subject is input to a receiving device for receiving a group of in-vivo images of the subject from a capsule endoscope introduced into organs of the subject without through the capsule endoscope to thereby the patient information of the subject.

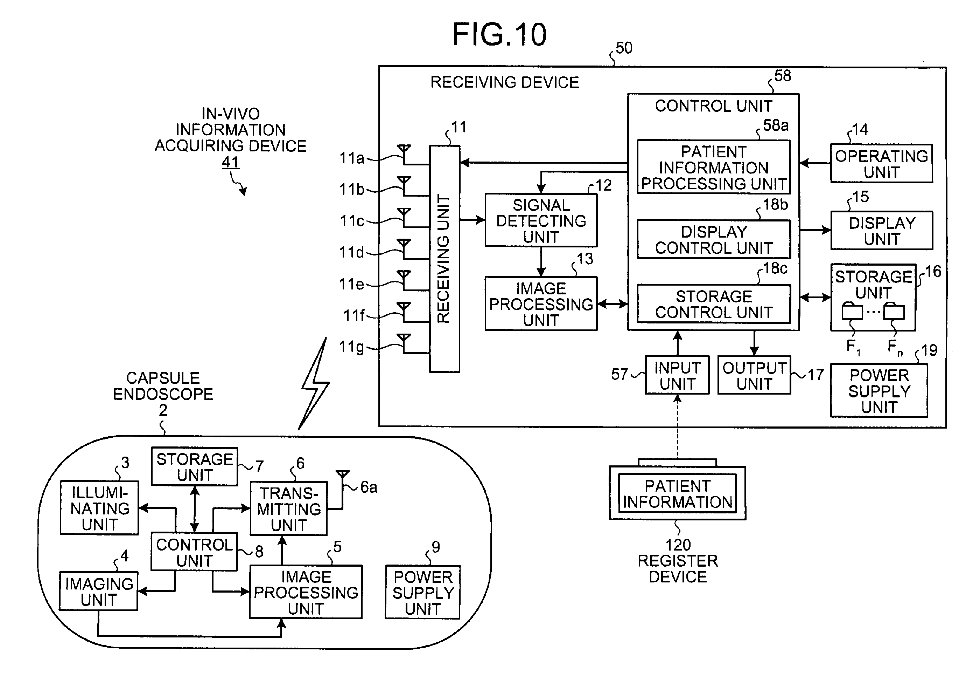

FIG. 10 is a block diagram schematically showing a configuration example of an in-vivo information acquiring device according to the third embodiment of the invention. As shown in FIG. 10, an in-vivo information acquiring device 41 according to the third embodiment has a receiving device 50 in place of the receiving device 10 of the in-vivo information acquiring device 1 according to the first embodiment described above. Note that a register device 120 is an external communication device for registering the patient information of a subject in the receiving device 50 and stores the patient information of the subject to which an in-subject inspection is executed. The register device 120 has such a structure that it can be detachably connected to the receiving device 50 and has a function for transmitting the patient information of the subject to the receiving device 50. Further, the capsule endoscope 2 of the in-vivo information acquiring device 41 is introduced into organs of a subject without capturing the specific image described above. The other configurations are the same as those of the first embodiment, and the same reference numerals are given to the same components.

The receiving device 50 obtains the patient information of the subject from the external register device 120 in place of that it receives the patient information of the subject (specifically, a specific image including the patient information of the subject) from the capsule endoscope 2 likewise the receiving device 10 of the in-vivo information acquiring device 1 according to the first embodiment described above. More specifically, the receiving device 50 obtains the patient information of the subject from the external register device 120 without through the capsule endoscope 2 and registers the obtained patient information. Further, the receiving device 50 sequentially receives in-vivo images of the subject from the capsule endoscope 2, causes the registered patient information to correspond to a group of the in-vivo images, and holds groups of the in-vivo images of a plurality of subjects by the patient information. The other functions of the receiving device 50 are the same as those of the receiving device 10 of the first embodiment described above.

The receiving device 50 has a control unit 58 in place of the control unit 18 of the receiving device 10 of the in-vivo information acquiring device 1 according to the first embodiment described above and further has an input unit 57 for inputting the patient information of a subject stored in the register device 120. Note that the image processing unit 13 of the receiving device 50 creates in-vivo images based on image signals of a frame unit obtained from the signal detecting unit 12 and sequentially transmits the created in-vivo images and a capsule ID to the control unit 58. The other configurations of the receiving device 50 are same as those of the receiving device 10 of the first embodiment described above, and the same reference numerals are given to the same components.

The input unit 57 acts as an input means for inputting the patient information of the subjects stored in the external register device 120 to the control unit 58. Specifically, the input unit 57 includes a communication interface which is connected to the register device 120 to execute a communication to it. The input unit 57 is detachably connected to the register device 120, obtains the patient information of the subjects stored in the register device 120, and inputs the obtained patient information to the control unit 58.

The control unit 58 controls the respective units (the receiving unit 11, the signal detecting unit 12, the image processing unit 13, the operating unit 14, the display unit 15, the storage unit 16, the output unit 17, and the input unit 57) of the receiving device 50 and controls signals input and output between the respective units. In this case, the control unit 58 obtains the patient information of the subjects stored in the external register device 120 through the input unit 57 and registers the obtained patient information in the storage unit 16. The other functions of the control unit 58 are the same as those of the control unit 18 of the receiving device 10 of the first embodiment described above.

The control unit 58 has a patient information processing unit 58a in place of the patient information processing unit 18a of the control unit 18 of the receiving device 10 described above and the display control unit 18b and the storage control unit 18c described above. The patient information processing unit 58a obtains the patient information of a subject input by the input unit 57 in place of extracting the patient information of the subject based on the specific image described above and newly registers the obtained patient information in the storage unit 16. The other functions of the patient information processing unit 58a is the same as those of the patient information processing unit 18a of the receiving device 10 of the first embodiment described above.

When the in-subject inspection is executed to a plurality of subjects, the control unit 58 having the above configuration repeats approximately the same processing procedure as that of steps S201 to S208 described above (refer to FIG. 4) and controls the storage unit 16 so that it stores groups of in-vivo images of the subjects by the patient information. In this case, the control unit 58 obtains the patient information of the subject input by the input unit 57 at step S202 described above. Each time the input unit 57 inputs the patient information of the subject, the control unit 58 registers the patient information of the subject obtained from the input unit 57 in the storage unit 16 and causes the registered patient information to correspond to a group of in-vivo images of a subject specified by the patient information.

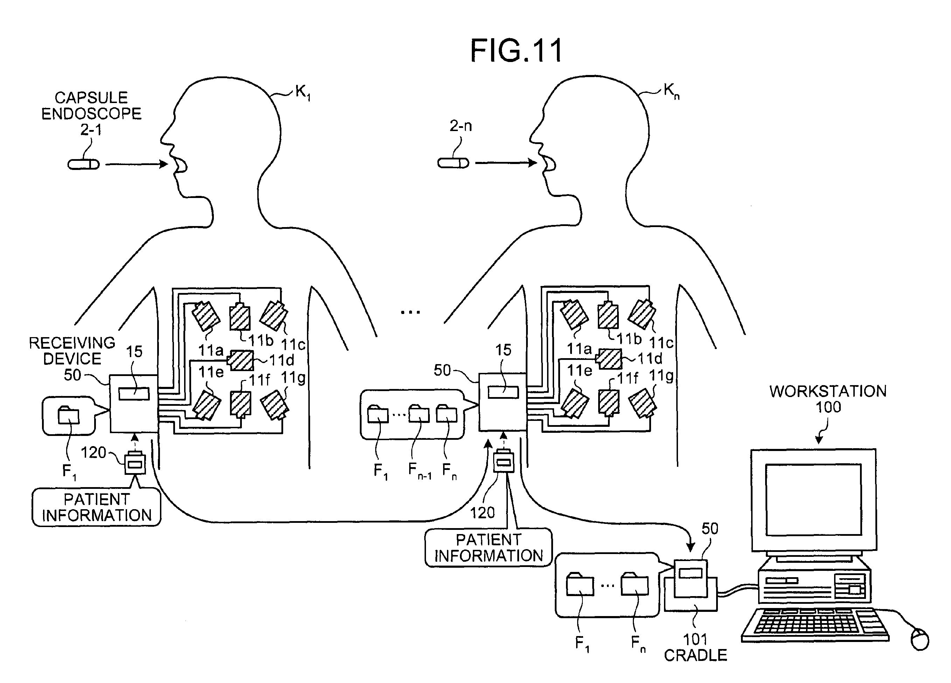

Next, an operation of the control unit 58 when it sequentially executes the in-subject inspection to subjects $K_1$, ..., $K_n$ will be specifically explained. FIG. 11 is a schematic view exemplifying a state that the in-subject inspection is executed to the subjects $K_1$, ..., $K_n$ using the in-vivo information acquiring device 41 according to the third embodiment.

When the in-vivo information acquiring device 41 according to the third embodiment is used, a user such as a doctor, a nurse, or the like repeats a job procedure approximately the same as that of steps S101 to S104 shown in FIG. 3 to the subjects $K_1$, ... $K_n$ and then executes the job procedure at steps S105 and S106 shown in FIG. 3 to thereby achieve the in-subject inspection to the subjects $K_1$, ..., $K_n$. Note that, when the in-vivo information acquiring device 41 according to the third embodiment is used, a means for registering the patient information of a subject to the receiving device 50 is different from that of the first embodiment. A difference between the first and the third embodiments in the in-subject inspection to subjects will be explained below referring to FIG. 11.

When register request information is displayed on the display unit 15 of the receiving device 50 worn on the n-th subject $K_n$ (n = 1, 2, 3, ...) at step S101 described above, the user such as the doctor, the nurse or the like registers the patient information of the subject $K_n$ in the receiving device 50 using the register device 120 in which the patient information of the subject $K_n$ is stored. In this case, the register device 120 is connected to the input unit 57 of the receiving device 50. The input unit 57 obtains the patient information of the subject $K_n$ from the register device 120 and inputs the obtained patient information to the control unit 58. The control unit 58 newly registers the patient information of the subject $K_n$ obtained through the input unit 57 in the storage unit 16 and newly creates a folder $F_n$ in the storage unit 16 using the newly registered patient information of the subject $K_n$ as a folder name.

The other inspection procedure (the inspection procedure at steps S102 to S106 shown in FIG. 3) is the same as that when the in-vivo information acquiring device 1 according to the first embodiment described above is used. More specifically, when the receiving device 50 obtains a group of in-vivo images of the n-th subject $K_n$, it holds groups of in-vivo images of the subjects $K_1$, ..., $K_n$ in a plurality of folders $F_1$, ..., $F_n$ of the storage unit 16 likewise the first embodiment described above. With this operation, the groups of the in-vivo images of the subjects $K_1$, ..., $K_n$ are caused to correspond to the respective patient information of the subjects $K_1$, ..., $K_n$ which as respective folder names of the folders $F_1$, ..., $F_n$.

Note that, although it is sufficient to realize the register device 120 described above using a portable recording medium having a predetermined connecting terminal such as an USB terminal and the like, it is further preferable that the register device 120 has a display unit such as an LCD and the like for displaying the patient information of a stored subject. With this arrangement, the patient information of the subjects held in the register device 120 can be easily checked, and the patient information of an erroneous subject can be prevented from being transmitted to the receiving device 50. Further, the register device 120 may have a function for storing the patient information of a plurality of subjects and transmitting the patient information of a desired subject selected from the subjects to the receiving device 50.

As explained above, in the third embodiment of the invention, the predetermined register device is connected to the input unit of the receiving device for receiving a group of in-vivo images of a subject captured by the capsule endoscope; the specific information of the subject is obtained by inputting the specific information of the subject stored in the register device by the input unit; and the obtained specific information of the subject is registered in the storage unit in which a group of in-vivo images captured by the capsule endoscope is stored. The other configurations of the third embodiment is the same as those of the first embodiment described above. As a result, the same operation/working effect as that of the first embodiment described above can be obtained as well as the in-vivo information acquiring device, which stores groups of in-vivo images of a plurality subjects by specific information can be realized by a simple configuration.

Modification of Third Embodiment

Next, a modification of the third embodiment of the invention will be explained. In the third embodiment described above, the external register device 120 is connected to the receiving device 50, and the patient information of the subject stored in the register device 120 is input to the receiving device 50. However, the modification of the third embodiment is configured such that an input unit such as an input key and the like is disposed to a receiving device for receiving a group of in-vivo images of a subject from a capsule endoscope introduced into organs of the subject, and the patient information of the subject is input to the receiving device by operating the input unit.

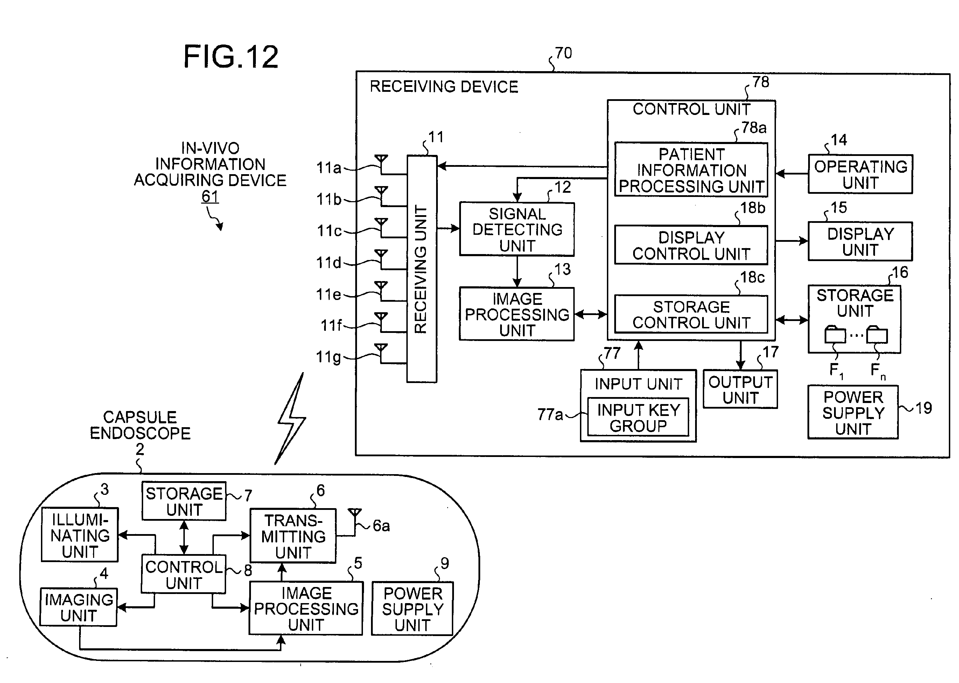

FIG. 12 is a block diagram schematically showing a configuration example of an in-vivo information acquiring device according to the modification of the third embodiment of the invention. As shown in FIG. 12, an in-vivo information acquiring device 61 according to the modification of the third embodiment has a receiving device 70 in place of the receiving device 50 of the in-vivo information acquiring device 41 according to the third embodiment described above. In the modification of the third embodiment, the patient information of a subject is input to the receiving device 70 without using the external register device 120 described above. The other configurations are the same as those of the third embodiment, and the same reference numerals are given to the same components.

The receiving device 70 obtains the patient information of a subject by being directly input with the patient information of the subject using the input key and the like in place of receiving it from the external register device 120 as in the receiving device 50 of the in-vivo information acquiring device 41 according to the third embodiment described above. The receiving device 70 registers the patient information of the subject obtained as described above, causes the registered patient information of the subject to correspond to a group of in-vivo images, and holds groups of in-vivo images of a plurality of subjects by the patient information. The other functions of the receiving device 70 are the same as those of the receiving device 50 of the third embodiment described above.

The receiving device 70 has a control unit 78 in place of the control unit 58 of the receiving device 50 of the in-vivo information acquiring device 41 according to the third embodiment described above and an input unit 77 in place of the input unit 57 to which the register device 120 is connected. The other configurations of the receiving device 70 are same as those of the receiving device 50 of the third embodiment described above, and the same reference numerals are given to the same components.

The input unit 77 acts as an input means for inputting the patient information of a subject, to which an in-subject inspection is executed, to the control unit 78. Specifically, the input unit 77 has an input key group 77a and inputs desired information to the control unit 78 by an input operation using the input key group 77a. The input unit 77 inputs the patient information of a desired subject, to which the in-subject inspection is executed, to the control unit 78.

The control unit 78 controls the respective units (the receiving unit 11, the signal detecting unit 12, the image processing unit 13, the operating unit 14, the display unit 15, the storage unit 16, the output unit 17, and the input unit 77) of the receiving device 70 and controls signals input and output between the respective units. In this case, the control unit 78 obtains the patient information of a subject input by the input unit 77 and registers the obtained patient information in the storage unit 16. The other functions of the control unit 78 are the same as those of the control unit 58 of the receiving device 50 of the third embodiment described above.

The control unit 78 has a patient information processing unit 78a in place of the patient information processing unit 58a of the control unit 58 of the receiving device 50 described above and the display control unit 18b and the storage control unit 18c described above. The patient information processing unit 78a obtains the patient information of the subject input by the input unit 77 by an input operation of the input key group 77a in place of acquiring the patient information of the subject stored in the register device 120 described above and newly registers the obtained patient information in the storage unit 16. The other functions of the patient information processing unit 78a are the same as those of the patient information processing unit 58a of the receiving device 50 of the third embodiment described above.

When an in-subject inspection is executed to a plurality of subjects, the control unit 78 having the above configuration repeats approximately the same processing procedure as that of steps S201 to S208 described above (refer to FIG. 4) and controls the storage unit 16 so that it stores groups of in-vivo images of the subjects by the patient information. In this case, the control unit 78 obtains the patient information of the subject input by the input unit 77 at step S202 described above. Each time the input unit 77 inputs the patient information of the subject, the control unit 78 registers the patient information of the subject obtained from the input unit 77 in the storage unit 16 and causes the registered patient information to correspond to a group of in-vivo images of a subject specified by the patient information.

The in-vivo information acquiring device 61 having the receiving device 70 and the capsule endoscope 2 can hold groups of in-vivo images of a plurality of the subjects by the patient information approximately likewise the in-vivo information acquiring device 41 according to the third embodiment described above. More specifically, when a user such as a doctor, a nurse, or the like uses the in-vivo information acquiring device 61, he or she repeats a job procedure approximately the same as that of steps S101 to S104 shown in FIG. 3 to the subjects $K_1$, ..., $K_n$ and then executes the job procedure at steps S105 and S106 shown in FIG. 3 to thereby achieve the in-subject inspection to the subjects $K_1$, ..., $K_n$.

Note that a means for acquiring the patient information of a subject of the in-vivo information acquiring device 61 is different from that of the in-vivo information acquiring device 41 according to the third embodiment described above. Specifically, when register request information is displayed on the display unit 15 of the receiving device 70 worn on the n-th subject $K_n$ (n = 1, 2, 3, ...) at step S101 described above, the user such as the doctor, the nurse, or the like inputs the patient information of the subject $K_n$ by operating the input key group 77a. In this case, the input unit 77 inputs the patient information of the subject $K_n$ to the control unit 78. The control unit 78 newly registers the patient information of the subject $K_n$ input by the input unit 77 to the storage unit 16 and newly creates a folder $F_n$, which uses the newly registered patient information of the subject $K_n$ as a folder name, in the storage unit 16. The other inspection procedure (the inspection procedure at steps S102 to S106 shown in FIG. 3) is the same as that when the in-vivo information acquiring device 41 according to the third embodiment described above is used.

As explained above, according to the modification of the third embodiment of the invention, the input unit such as the input key and the like is disposed to the receiving device for receiving a group of in-vivo images of a subject captured by the capsule endoscope; the specific information of the subject is obtained by that the input unit inputs the specific information of the subject; and the obtained specific information of the subject is registered in the storage unit which in which a group of in-vivo images captured by the capsule endoscope are stored. The other configurations of the modification is the same as those of the third embodiment described above. Therefore, the specific information of a desired subject can be input to the receiving device without using an external register device and the like and the same operation/working effect as that of the third embodiment described above can be obtained as well as a troublesome work for carrying an external device in which the specific information of a subject is stored can be omitted.

Note that, in the first to the third embodiments and the modification of the invention, although the in-vivo information acquiring device having the capsule endoscope for capturing a group of in-vivo images of a subject is exemplyfied as an example of the in-vivo information of the subject, it is not limited thereto and may be an in-vivo information acquiring device having an in-subject introduction device, which is introduced into organs of a subject and obtains the in-vivo information of a subject in place of the capsule endoscope for capturing the group of the in-vivo images. In this case, the in-subject introducing device may obtain in-vivo information, for example, temperature information, pH information, living body tissue information, or the like in organs.

Further, in the first to the third embodiments and the modification of the invention, although the in-vivo information of the subject (for example, a group of in-vivo images) is caused to correspond to the patient information (a patient ID, a patient name, a gender, a birth day, and the like) of the subject, it is not limited thereto, and the specific information of the subject may be the in-vivo information of the subject. The specific information of the subject may be, for example, a face image of the subject, and the face image may be caused to correspond to a group of in-vivo images. In this case, it is sufficient to arrange the face image of the subject as, for example, a thumbnail image and to add it to a folder and to store a group of in-vivo images of the subject in the folder.

Further, in the first embodiment of the invention, a specific image including the patient information of a subject is obtained by using the optical information recording medium to which optical information corresponding to the patient information of the subject is recorded and capturing the optical information recording medium. However, the first embodiment is not limited thereto and the specific information may obtained by using a medium, to which the patient information of a subject is recorded such as a medical record, a name plate, or the like of the subject and capturing the medium.

Further, in the first to the third embodiments and the modification of the invention, register request information for requesting to newly register the specific information of a subject is displayed when the receiving device is started and when the acquisition of a group of in-vivo images of a subject has been finished. However, the first to the third embodiments and the modification are not limited thereto, and the register request information may be displayed when it is instructed to start to store in-vivo images of the subject. In this case, it is sufficient that the operating unit 14 of the receiving device described above instructs the control unit to start to store the in-vivo images, and each time the instruction is issued, the display unit 15 displays the register request information. Thereafter, when the specific information of the subject is newly registered in the storage unit 16, the display unit 15 may display register finish information for a predetermined time in place of the register request information and display the registered specific information in place of the register finish information after the predetermined time passes.

Further, in the first to the third embodiments and the modification of the invention, when a predetermined time passes after the specific information of the subject is newly registered in the storage unit 16, the acquisition of a group of in-vivo images of the subject is finished. However, the first to the third embodiments and the modification are not limited thereto, and the acquisition of the group of the in-vivo images of the subject may be finished when the receiving device, which receives the group of the in-vivo images of the subject through the receiving antennas, is disconnected from the connectors connecting therebetween. In the first to the third embodiments and the modification, the in-vivo information acquiring device be provided may further include a position detector for detecting a position of the capsule endoscope introduced in the organ of the subject, and when the position of the capsule endoscope detected by the position detector is a position where it passes in an desired organ, it may obtain a group of the in-vivo images of the subject.

Further, in the second embodiment of the invention, the RFID tag 23 for receiving the specific information of a subject transmitted from the RFID writer 110 is contained in the capsule endoscope. However, the second embodiment is not limited thereto, and the means for receiving the patient information of the subject may a means for receiving the specific information of the subject by wirelessly communicating with the external device that holds the specific information of the subject and may be, for example, a receiving means for receiving the specific information of the subject by transmitting and receiving a predetermined radio wave, infrared rays, or the like.

Further, in the third embodiment of the invention, the register device 120 including the specific information of a subject is connected to the receiving device 50 through a connector, and the receiving device 50 obtains the specific information of the subject by communicating with the register device 120 through a cable. However, the third embodiment is not limited thereto and the register device 120 may transmit the patient information of the subject to the receiving device 50 by executing a wirelessly communication (for example, an infrared ray communication or an RFID communication) to the receiving device 50.

Further, the register device 120 may has an input means such as an input key, a touch panel, or the like and may transmit the specific information of a subject input thereto by the input means to the receiving device 50. Further, when the receiving device 50 is driven by power of a battery, a switch for instructing to start a radio communication to the register device 120 having a wireless communication function may be disposed to the receiving device 50, and only when it is instructed to start the radio communication by depressing the switch, the register device 120 and the receiving device 50 may start the radio communication to obtain the specific information of a subject.

Further, the workstation 100 may be connected to the receiving device 50 through a cradle and the like in place of the register device 120, and the receiving device 50 may obtain the specific information of a subject from the workstation 100 by an information communication between the workstation 100 and the receiving device 50.

Further, in the first to the third embodiments and the modification of the invention, the groups of in-vivo images of the subjects are stored in the storage unit 16 contained in the receiving device. However, the first to the third embodiments and the modification are not limited thereto, and the storage unit 16 may be realized using a portable recording medium and a storage unit to which the storage medium is detachably attached, and the groups of the in-vivo images of the subjects may be stored in the recording medium attached to the storage unit in each specific information. In this case, the groups of the in-vivo images of the subjects obtained by the receiving device are captured by the workstation from the receiving device through the recording medium.

INDUSTRIAL APPLICABILITY

As described above, the in-vivo information acquiring device according to the invention is useful to an in-subject inspection for acquiring in-vivo information such as an in-vivo image and the like of a subject, and in particularly can store groups of n-vivo information obtained from a plurality of subjects by specific information of the subjects. As a result, the in-vivo information acquiring device is suitable for an in-vivo information acquiring device which can smoothly execute an in-subject inspection to a plurality of subjects.

**Claims**

1. An in-vivo information acquiring device for acquiring in-vivo information on a plurality of subjects, comprising:

an information acquiring unit configured to acquire specific information for specifying the subjects;
a storage unit configured to store therein the in-vivo information on the subjects; and
a control unit configured to execute control of registering the specific information acquired by the information acquiring unit in the storage unit and causing the registered specific information to correspond to the in-vivo information on a subject specified by the specific information so as to store the in-vivo information on the subjects by the specific information.

2. The in-vivo information acquiring device according to claim 1, wherein
the information acquiring unit is an imaging unit for capturing a specific image including the specific information, and
the control unit executes control of registering the specific information included in the specific image captured by the imaging unit in the storage unit and causing the registered specific information to correspond to the in-vivo information on a subject specified by the specific information so as to store the in-vivo information on the subjects by the specific information.

3. The in-vivo information acquiring device according to claim 2, wherein
the in-vivo information is an in-vivo image obtained by imaging inside of an organ of the subject, and
the imaging unit images the specific image and further images the in-vivo image.

4. The in-vivo information acquiring device according to claim 2, wherein
the specific image is living body information of the subject.

5. The in-vivo information acquiring device according to claim 1, wherein
the information acquiring unit is a receiving unit for receiving the specific information by executing a radio communication, and
the control unit executes control of registering the specific information received by the receiving unit and causing the registered specific information to correspond to the in-vivo information on a subject specified by the specific information so as to store the in-vivo information on the subjects by the specific information.

**6.** The in-vivo information acquiring device according to claim 1, wherein
the information acquiring unit is an input unit for inputting the specific information to the control unit, and
the control unit executes control of registering the specific information input by the input unit to the storage unit and causing the registered specific information to correspond to the in-vivo information on a subject specified by the specific information so as to store the in-vivo information on the subjects by the specific information.

**7.** The in-vivo information acquiring device according to claim 1, further comprising a display unit for displaying register request information for requesting to newly register the specific information, wherein
the control unit executes a control for displaying the register request information before newly registering the specific information in the storage unit.

**8.** The in-vivo information acquiring device according to claim 1, wherein
the control unit determines, for each piece of the in-vivo information, whether acquisition of the in-vivo information to be stored in the storage unit has been finished and causes, during a period from a time at which specific information is newly registered in the storage unit to a time at which the control unit determines that the acquisition of the in-vivo information of a subject specified by the newly registered specific information has been finished, a series of the in-vivo information stored in the storage unit to correspond to the newly registered specific information.

**9.** The in-vivo information acquiring device according to claim 8, further comprising an instruction unit for instructing to finish to store the in-vivo information on the subject, wherein
when the instruction unit instructs to finish the storage, the control unit determines that the acquisition of the in-vivo information of the subject specified by the newly registered specific information has been finished and causes, during a period from a time at which specific information is newly registered in the storage unit to a time at which the instruction unit instructs to finish the storage, a series of the in-vivo information stored in the storage unit to correspond to the newly registered specific information.

**10.** The in-vivo information acquiring device according to claim 2, wherein
the specific image is an optical information recording medium on which optical information corresponding to the subject is recorded.

**11.** The in-vivo information acquiring device according to claim 1, wherein
the control unit determines that acquisition of the in-vivo information of a subject specified by the newly registered specific information has been finished based on an arbitrarily set period of time after specific information is newly registered in the storage unit and causes a series of in-vivo information stored in the storage unit to correspond to the newly registered specific information during a period until the determination is made.

**12.** The in-vivo information acquiring device according to claim 1, wherein
the control unit determines that acquisition of the in-vivo information on a subject specified by specific information has been finished based on at least one of color information, luminance information, and space-frequency information of the in-vivo information of a subject specified by the newly registered specific information after specific information is registered in the storage unit, and causes a series of in-vivo information stored in the storage unit to correspond to the newly registered specific information during a period until the determination is made.

**13.** The in-vivo information acquiring device according to claim 1, wherein
the control unit determines, by inputting the in-vivo information of a subject specified by the newly registered specific information from the outside after specific information is registered in the storage unit, that acquisition of the in-vivo information on a subject specified by specific information has been finished, and causes a series of in-vivo information stored in the storage unit to correspond to the newly registered specific information during a period until the determination is made.

# FIG.1

IN-VIVO
INFORMATION
ACQUIRING DEVICE
1

RECEIVING DEVICE 10

CONTROL UNIT 18

PATIENT
INFORMATION
PROCESSING
UNIT 18a

OPERATING
UNIT 14

RECEIVING UNIT 11

SIGNAL
DETECTING
UNIT 12

DISPLAY
CONTROL UNIT 18b

DISPLAY
UNIT 15

IMAGE
PROCESSING
UNIT 13

STORAGE
CONTROL UNIT 18c

STORAGE
UNIT 16
F₁ ... Fₙ

OUTPUT
UNIT 17

POWER
SUPPLY
UNIT 19

CAPSULE
ENDOSCOPE
2

ILLUMI-
NATING
UNIT 3

STORAGE
UNIT 7

TRANS-
MITTING
UNIT 6
6a

CONTROL
UNIT 8

IMAGING
UNIT 4

IMAGE
PROCESSING
UNIT 5

POWER
SUPPLY
UNIT 9

EP 2 165 639 A1

# FIG.2

OPTICAL INFORMATION
RECORDING MEDIUM
A

PATIENT
INFORMATION

CAPSULE
ENDOSCOPE
2

4

# FIG.3

```
                    ┌─────────────┐
                    │    START    │
                    └──────┬──────┘
                           │
                           ▼ ◄─────────────────────────┐
        ┌──────────────────────────────┐               │
        │     REGISTER OF PATIENT       │ ~S101         │
        │        INFORMATION            │               │
        └───────────────┬──────────────┘               │
                        │                               │
                        ▼                               │
        ┌──────────────────────────────┐               │
        │     SWALLOW OF CAPSULE        │ ~S102         │
        │        ENDOSCOPE              │               │
        └───────────────┬──────────────┘               │
                        │                               │
                        ▼                               │
        ┌──────────────────────────────┐               │
        │     INSPECTION OF SUBJECT     │ ~S103         │
        └───────────────┬──────────────┘               │
                        │                               │
                        ▼           S104                │
                ◇─────────────────────◇                 │
               ╱          IS           ╲   YES          │
              ◇   INSPECTION OF NEXT    ◇───────────────┘
               ╲  SUBJECT EXECUTED?    ╱
                ◇─────────────────────◇
                        │ NO
                        ▼
        ┌──────────────────────────────┐
        │  DOWNLOAD OF IN-VIVO IMAGES   │ ~S105
        └───────────────┬──────────────┘
                        │
                        ▼
        ┌──────────────────────────────┐
        │     DIAGNOSIS OF SUBJECT      │ ~S106
        └───────────────┬──────────────┘
                        │
                        ▼
                 ┌─────────────┐
                 │     END     │
                 └─────────────┘
```

# FIG.4

```
                          ┌─────────────┐
                          │    START    │
                          └─────────────┘
                                 │
                                 ▼
        ┌──────────────────────────────────────┐
        │ DISPLAY OF REGISTER REQUEST OF        │ ～S201
        │ PATIENT INFORMATION                   │
        └──────────────────────────────────────┘
                                 │
                                 ▼
        ┌──────────────────────────────────────┐
        │ ACQUISITION OF PATIENT                │ ～S202
        │ INFORMATION                           │
        └──────────────────────────────────────┘
                                 │
                                 ▼
        ┌──────────────────────────────────────┐
        │ REGISTER OF PATIENT                   │ ～S203
        │ INFORMATION                           │
        └──────────────────────────────────────┘
                                 │
                                 ▼
        ┌──────────────────────────────────────┐
        │ CREATION OF NEW FOLDER OF             │ ～S204
        │ EACH PIECE OF PATIENT                 │
        │ INFORMATION                           │
        └──────────────────────────────────────┘
                                 │
                                 ▼
        ┌──────────────────────────────────────┐
        │ ACQUISITION OF IN-VIVO IMAGE          │ ～S205
        └──────────────────────────────────────┘
                                 │
                                 ▼
        ┌──────────────────────────────────────┐
        │ STORAGE OF IN-VIVO IMAGE IN NEW       │ ～S206
        │ FOLDER                                │
        └──────────────────────────────────────┘
                                 │
                                 ▼
                    ┌────────────────────┐  S207
                    │       HAS          │
                    │   ACQUISITION OF   │
                    │ IN-VIVO IMAGE OF CURRENT │  NO
                    │  SPECIMEN BEEN     │
                    │    FINISHED?       │
                    └────────────────────┘
                             │ YES
                             ▼
        ┌──────────────────────────────────────┐
        │ END OF STORAGE OF IN-VIVO IMAGE       │ ～S208
        │ TO NEW FOLDER                         │
        └──────────────────────────────────────┘
```

# FIG.5

EP 2 165 639 A1

# FIG.6

IN-VIVO INFORMATION
ACQUIRING DEVICE
21

**RECEIVING DEVICE** 30

**RECEIVING UNIT** 11

11a
11b
11c
11d
11e
11f
11g

RECEIVING UNIT

RFID
WRITER 110

SIGNAL
DETECTING
UNIT 12

IMAGE
PROCESSING
UNIT 33

INFORMATION
EXTRACTING
UNIT 33a

**CONTROL UNIT** 38

PATIENT
INFORMATION
PROCESSING UNIT 38a

DISPLAY CONTROL
UNIT 18b

STORAGE
CONTROL UNIT 38c

OPERATING
UNIT 14

DISPLAY
UNIT 15

STORAGE
UNIT 16

$F_1$ ··· $F_n$

OUTPUT
UNIT 17

POWER
SUPPLY
UNIT 19

CAPSULE
ENDOSCOPE
22

STORAGE
UNIT 7

RFID TAG 23 23a

ILLUMI-
NATING
UNIT 3

CONTROL
UNIT 28

TRANS-
MITTING
UNIT 6 6a

IMAGING
UNIT 4

IMAGE
PROCESSING
UNIT 25

ADDITION
PROCESSING
UNIT 25a

POWER
SUPPLY UNIT 9

EP 2 165 639 A1

31

# FIG.7

| IMAGE DATA | PATIENT INFOR- MATION |

| IMAGE DATA | PATIENT INFOR- MATION |

# FIG.8

START

DISPLAY OF PATIENT INFORMATION REGISTER REQUEST —S301

ACQUISITION OF PATIENT INFORMATION AND IN-VIVO IMAGE —S302

REGISTER OF PATIENT INFORMATION —S303

CREATION OF NEW FOLDER OF EACH PIECE OF PATIENT INFORMATION —S304

STORAGE OF IN-VIVO IMAGE —S305

ACQUISITION OF SUBSEQUENT IN-VIVO IMAGE AND PATIENT INFORMATION —S306

COMPARISON OF SUBSEQUENT PATIENT INFORMATION WITH PATIENT INFORMATION IN FOLDER —S307

STORAGE OF IN-VIVO IMAGE IN FOLDER THAT AGREES WITH PATIENT INFORMATION —S308

HAS ACQUISITION OF IN-VIVO IMAGE OF CURRENT SPECIMEN BEEN FINISHED? S309

NO

YES

# FIG.9

EP 2 165 639 A1

# FIG.10

RECEIVING DEVICE 50

IN-VIVO INFORMATION ACQUIRING DEVICE 41

CONTROL UNIT 58

11a 11b 11c 11d 11e 11f 11g 11

RECEIVING UNIT

SIGNAL DETECTING UNIT 12

IMAGE PROCESSING UNIT 13

PATIENT INFORMATION PROCESSING UNIT 58a

DISPLAY CONTROL UNIT 18b

STORAGE CONTROL UNIT 18c

OPERATING UNIT 14

DISPLAY UNIT 15

STORAGE UNIT 16
F₁ ... Fₙ

INPUT UNIT 57

OUTPUT UNIT 17

POWER SUPPLY UNIT 19

PATIENT INFORMATION

REGISTER DEVICE 120

CAPSULE ENDOSCOPE 2

STORAGE UNIT 7

ILLUMI-NATING UNIT 3

TRANS-MITTING UNIT 6
6a

IMAGING UNIT 4

CONTROL UNIT 8

IMAGE PROCESSING UNIT 5

POWER SUPPLY UNIT 9

EP 2 165 639 A1

# FIG.11

EP 2 165 639 A1

# FIG.12

EP 2 165 639 A1

IN-VIVO
INFORMATION
ACQUIRING DEVICE
61

RECEIVING DEVICE 70

CONTROL UNIT 78

11

11a
11b
11c
11d
11e
11f
11g

RECEIVING UNIT 11

SIGNAL DETECTING UNIT 12

IMAGE PROCESSING UNIT 13

PATIENT INFORMATION PROCESSING UNIT 78a

DISPLAY CONTROL UNIT 18b

STORAGE CONTROL UNIT 18c

OPERATING UNIT 14

DISPLAY UNIT 15

STORAGE UNIT 16
F₁ ... Fₙ

INPUT UNIT 77
INPUT KEY GROUP 77a

OUTPUT UNIT 17

POWER SUPPLY UNIT 19

CAPSULE ENDOSCOPE 2

STORAGE UNIT 7

TRANS-MITTING UNIT 6
6a

ILLUMI-NATING UNIT 3

CONTROL UNIT 8

IMAGING UNIT 4

IMAGE PROCESSING UNIT 5

POWER SUPPLY UNIT 9

<table>
<tr><td colspan="3" align="center">**INTERNATIONAL SEARCH REPORT**</td><td>International application No.<br>PCT/JP2008/060217</td></tr>
</table>

| A. CLASSIFICATION OF SUBJECT MATTER |
|---|
| *A61B1/00*(2006.01)i, *A61B1/04*(2006.01)i, *A61B5/07*(2006.01)i |

According to International Patent Classification (IPC) or to both national classification and IPC

| B. FIELDS SEARCHED |
|---|
| Minimum documentation searched (classification system followed by classification symbols)<br>A61B1/00, A61B1/04, A61B5/07 |

Documentation searched other than minimum documentation to the extent that such documents are included in the fields searched
Jitsuyo Shinan Koho        1922-1996   Jitsuyo Shinan Toroku Koho   1996-2008
Kokai Jitsuyo Shinan Koho   1971-2008   Toroku Jitsuyo Shinan Koho   1994-2008

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X<br>Y | JP 2005-296186 A  (Olympus Corp.),<br>27 October, 2005 (27.10.05),<br>Full text; all drawings<br>& US 2007/0081077 A1    & WO 2005/099555 A<br>& CN 1937950 A | 1-10,13<br>11,12 |
| X | JP 2006-61470 A  (Olympus Corp.),<br>09 March, 2006 (09.03.06),<br>Full text; all drawings<br>(Family: none) | 1,6,8,9,13 |
| Y | JP 2005-304512 A  (Olympus Corp.),<br>04 November, 2005 (04.11.05),<br>Full text; all drawings<br>(Family: none) | 11 |

| ☒ | Further documents are listed in the continuation of Box C. | ☐ | See patent family annex. |
|---|---|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>26 August, 2008 (26.08.08) | Date of mailing of the international search report<br>09 September, 2008 (09.09.08) |
|---|---|
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

International application No.

PCT/JP2008/060217

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2007-68568 A (Olympus Corp.),<br>22 March, 2007 (22.03.07),<br>Full text; all drawings<br>& WO 2007/026891 A1 | 12 |

Form PCT/ISA/210 (continuation of second sheet) (April 2007)

**EP 2 165 639 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP 2005296186 A